# EUROPEAN PATENT SPECIFICATION

(11) **EP 3 027 058 B1**
(45) Date of publication and mention of the grant of the patent: **15.07.2020**
(21) Application number: 14832924.6
(22) Date of filing: 31.07.2014
(51) Int. Cl.: A23L 33/00, A23L 33/10, A23L 2/84, A61K 35/741

(54) **ENCAPSULATED FUNCTIONAL FOOD COMPOSITIONS**
VERKAPSELTE FUNKTIONELLE NAHRUNGSMITTELZUSAMMENSETZUNGEN
COMPOSITIONS D'ALIMENT FONCTIONNEL ENROBÉ

(30) Priority: 31.07.2013 US 201361860586 P; 31.07.2013 US 201361860609 P; 31.07.2013 US 201361860652 P; 31.07.2013 US 201361860629 P; 31.07.2013 US 201361860625 P; 31.07.2013 US 201361860591 P; 31.07.2013 US 201361860623 P; 31.07.2013 US 201361860595 P; 31.07.2013 US 201361860617 P; 31.07.2013 US 201361860599 P; 04.03.2014 US 201461947803 P
(43) Date of publication of application: 08.06.2016
(73) Proprietor: Incredible Foods, Inc., Boston, MA 02136 (US)
(72) Inventor: HORN, Gregory T., Lighthouse Point, Florida 33064 (US)
(74) Representative: V.O.
(86) International application number: PCT/US2014/049066
(87) International publication number: WO 2015/017625

(56) References cited:
- WO-A2-2012/008994
- KR-A- 20090 116 517
- US-A1- 2003 138 520
- US-A1- 2004 161 422
- US-A1- 2005 112 235
- US-A1- 2006 093 592
- US-A1- 2007 048 295
- US-A1- 2010 028 449
- US-A1- 2012 015 075
- US-A1- 2013 115 334
- US-A1- 2013 209 614
- RUSSO ET AL.: 'Beta-Glucans Improve Growth, Viability and Colonization of Probiotic Microorganisms.' INTERNATIONAL JOURNAL OF MOLECULAR SCIENCE vol. 13, 2012, pages 6026 - 6039, XP055315026

## Description

### TECHNICAL FIELD

This disclosure relates to functional food compositions, membrane encased edible compositions, and more particularly to fully encased, functional food compositions.

### BACKGROUND

Functional foods provide nutritional components that are important for health maintenance. These food compositions contain compounds that are biologically active or bioavailable, such as probiotics, amino acids, multivitamins, and antioxidants, and often are found to be useful for the treatment of disease and disorders or the maintenance of normal health states. However, delivery systems for bioavailable compounds often can have insufficient viability, resulting in suboptimal bioactive quantities and bioavailability of these compounds. Many functional compounds are beneficial to hosts only when consumed in sufficient quantities. Therefore, consumers continually seek functional food compositions with enhanced effectiveness.

US 2010/0028449 discloses microcapsules comprising probiotics, which may be used in combination with a prebiotic carrier such as fermented milk. KR 20090116517 discloses double encapsulated probiotics which may be used to supplement milk, said milk being itself supplemented with the prebiotic inulin.

### SUMMARY

This disclosure relates to a variety of functional food compositions. Functional food compositions are designed to deliver sufficient quantities and quality of bioactive and bioavailable compositions using edible or potable substances encapsulated in an edible membrane matrix. The encapsulated composition provides certain advantages in its transport, handling and consumption. The edible membrane matrix can be constructed to include edible particles that impart advantages to the performance of the membrane matrix, provide functional nutrition, health and well-being benefits, therapeutic treatment to the consumer, and/or enhance consumer gustatory experience. In particular aspects, the functional food composition provides nutritive and health benefits for disease treatment and prevention, and health maintenance through the incorporation of certain biologically active compounds in the edible substance and/or as particles in the membrane matrix.

According to the invention, which is defined by the claims, it is provided herein is a functional food composition comprising an edible or potable substance, a cross-linked edible membrane matrix fully encapsulating the edible or potable substance, a probiotic, and a prebiotic, with the proviso that the edible or potable substance and the matrix do not both contain the probiotic or both contain the prebiotic, wherein the matrix comprises the prebiotic and the edible or potable substance component comprises the probiotic, or wherein the matrix comprises the probiotic and the edible or potable substance comprises the prebiotic. The functional food composition can comprise a probiotic concentration from about 1.0 to about 100 billion colony forming units in an individual edible transport vessel, and the probiotic can be an organism selected from the genera group consisting of *Aspergillus, Bacillus, Bacteroides, Bifidobacterium, Brettanomyces, Enterococcum, Kluyveromyces, Lactobacillus, Lactococcus, Leuconostoc, Pediococcus, Propionibacterium, Saccharomyces, Shewanella, Streptococcus, Torulaspora, Vagococcus,* and any derivatives, strains, and combinations thereof.

The invention also relates to a method of preparing a functional food composition, comprising the steps of:
a) providing an edible or potable substance, an edible polymer, a probiotic, and prebiotic;
b) combining the edible or potable substance with the probiotic or the prebiotic;
c) encapsulating the edible substance with the probiotic or the prebiotic in an edible matrix that comprises the edible polymer and the probiotic or the prebiotic;
with the proviso that the edible or potable substance and the matrix do not both contain the probiotic or both contain the prebiotic.

The prebiotic of the functional food composition can be one of the group consisting of galacto-oligosaccharides, inulin, oligofructose, isomalto-oligosaccharides, lactulose, lactosucrose, transgalacto-oligosaccharides, soybean oligosaccharides, tagatose, xylo-oligosaccharides, and combinations thereof. The prebiotic concentration can be from about 1.0 to about 30.0 grams in an individual edible transport vessel.

The functional food composition can comprise additional edible particles in the cross-linked matrix. The prebiotic, the probiotic, and/or additional edible particles can provide enhanced performance to the matrix.

The prebiotic, the probiotic, and/or additional edible particles can comprise a size having a volume mean distribution between about 0.1 microns and about 1.0 microns, between about 0.1 microns and about 10.0 microns, between about 0.1 microns and about 100.0 microns, between about 0.1 microns and about 1.0 millimeters, or between about 0.1 and about 3 millimeters.

The functional food composition can be used for the normalization of gastro-intestinal flora in a subject, for the prevention and treatment of a gastrointestinal disorder, for the prevention and treatment of a systemic disorder, to improve immune function, for improved digestion, and to decrease intestinal gas production.

Described is a method for maintaining or improving digestive health by administering to an individual in need thereof a functional food composition as described herein. The functional food composition can be administered in an amount sufficient to increase the gastrointestinal microflora concentration of an individual. The functional food composition can be administered in an amount sufficient to increase the fecal microflora concentration of an individual. The functional food composition can be administered in an amount sufficient to maintain normal gastrointestinal microflora concentration of an individual. The functional food composition can be administered in an amount sufficient to provide relief from gastrointestinal bloating of an individual.

Described is a method for the prevention and treatment of a gastrointestinal disorder by administering to an individual in need thereof a functional food composition as described herein, where the gastrointestinal disorder comprises at least one of the group comprising diarrhea, Traveler's Diarrhea, antibiotic-associated diarrhea, inflammatory bowel disease, excessive stomach acid, dyspepsia, constipation, irritable bowel syndrome, Crohn's Disease, lactose intolerance, and pathogenic infection.

The functional food can be administered in an amount sufficient to prevent or treat the establishment of opportunistic gastrointestinal pathogens.

Described is a method for the treatment of a systemic disorder by administering to an individual in need thereof a functional food composition as described herein, where the systemic disorder comprises at least one of the group consisting of elevated cholesterol, hypertension, acid reflux disease, colon cancer, and obesity.

Described is a method for improving immune response by administering to an individual in need thereof a functional food composition as described herein, where improving immune response further comprises lowering the severity of at least one of the group consisting of diarrhea, Traveler's Diarrhea, antibiotic-associated diarrhea, inflammatory bowel disease, excessive stomach acid, dyspepsia, constipation, irritable bowel syndrome, Crohn's Disease, lactose intolerance, and pathogenic infection.

This disclosure also relates to the delivery of bioflavonoids, carotenes and elastic tissue components using edible or potable substances encapsulated in an edible membrane matrix. In particular aspects, the functional food provides functionally nutritive benefits for integument disorder prevention and health, beauty and wellness maintenance through the incorporation of certain biologically active compounds in the edible substance and/or as particles in the membrane matrix.

Described herein is a functional food composition and method for making, comprising an edible or potable substance, a cross-linked matrix encapsulating the edible or potable substance, the cross-linked matrix comprising an edible polymer, and an integument health compound comprising a bioflavonoid, a carotene, and an elastic tissue component.

The functional food composition can include a bioflavonoid derived from at least one of the group consisting of *Camellia sinensis, Theobroma cacao,* varieties thereof, strains thereof, cultivars thereof, and combinations thereof.

The functional food composition can include bioflavonoid selected from the group consisting of esveratrol, quercetin, rutin, catechin, epicatechin, proanthocyanidin, cocoaflavanol, and combinations thereof.

The functional food composition can include an essential fatty acid selected from the group consisting of α-linolenic acid, eicosapentanoic acid, docosahexaenoic acid, and combinations thereof.

The functional food composition can include a carotene selected from the group consisting of α-carotene, β-carotene, γ-carotene, δ-carotene, ε-carotene, lycopene, and combinations thereof.

The functional food composition can include a connective tissue component selected from the group consisting of collagen, elastin, and combinations thereof.

The functional food composition can comprise additional edible particles in the cross-linked matrix. The cardiovascular health compound and/or additional edible particles can provide enhanced performance to the matrix.

The functional food composition of the integument health compound and/or additional edible particles can have a size with a volume mean distribution between about 0.1 microns and about 1.0 microns, between about 0.1 microns and about 10.0 microns, between about 0.1 microns and about 100.0 microns, between about 0.1 microns and about 1.0 millimeters, or between about 0.1 and about 3 millimeters.

Described herein is a method of preparing a functional food composition, comprising the steps of providing an edible or potable substance, an edible unpolymerized matrix, and an integument health compound comprising a bioflavonoid, a carotene, and an elastic tissue component, combining the edible or potable substance and/or the edible unpolymerized matrix with the integument health compound, and encapsulating the edible substance with the edible matrix. The method can further comprise the step of polymerizing the edible matrix.

The functional food composition can be used for maintenance of skin, for the maintenance of nails, for the maintenance of hair, for the prevention and treatment of dry skin, for the prevention and treatment of skin wrinkles, for the prevention and treatment of skin spots, for the prevention and treatment of brittle nails, for the prevention and treatment of hair loss.

Described is a method for the maintenance of skin health by administering to an individual in need thereof a functional food composition as described herein.

Described is a method for the maintenance of hair health by administering to an individual in need thereof a functional food composition as described herein.

Described is a method for the maintenance of nail health by administering to an individual in need thereof a functional food composition as described herein.

Described is a method for the prevention and treatment of hair loss by administering to an individual in need thereof a functional food composition as described herein.

Described is a method for the prevention and treatment of brittle nails by administering to an individual in need thereof a functional food composition as described herein.

Described is a method for the prevention and treatment of skin wrinkles by administering to an individual in need thereof a functional food composition as described herein.

Described is a method for the prevention and treatment of skin spots by administering to an individual in need thereof a functional food composition as described herein.

This disclosure also relates to the delivery of compounds useful for weight loss using edible or potable substances encapsulated in an edible membrane matrix. In particular aspects, the functional food provides functionally nutritive benefits for disease prevention and health wellness through the incorporation of certain biologically active compounds in the edible substance and/or as particles in the membrane matrix.

Described herein is a functional food composition and method for making, comprising an edible or potable substance, a cross-linked matrix encapsulating the edible or potable substance, and particles for weight loss. The functional food composition can include a weight loss compound selected from the group consisting of an appetite suppressant, fat uptake inhibitors, gastrointestinal fillers, and thermogenic compounds.

The functional food composition can include a weight loss compound selected from the group consisting of thylakoids, thylakoid extracts, and combinations thereof.

The functional food composition can include at least one of the group consisting of a vitamin, a mineral, an amino acid, an antioxidant, an anti-inflammatory agent, and an essential fatty acid.

The functional food composition can include at least one of the group consisting proteinase inhibitor II, green coffee bean extract, chlorogenic acid, green tea leaf extract, polyphenols, ashwagandha extract, xanthum gum, and pinolenic acid, hoodia, chitosan, chromium picolinate, conjugated linoleic acid, glucomannan, green tea extract, guar gum, guarana, guggal, senna, ephedra, bitter orange, fucoxanthin, white bean extract, vitamin D, human chorionic gonadotropin, resveratrol, capsaicin, chia, hoodia, L-carnitine, raspberry ketones, banana leaf, red clover, ginger, almonds, acai berry, flax seeds, leucine, and lipodrene.

The functional food composition can comprise additional edible particles in the cross-linked matrix. The edible particles weight loss and/or additional edible particles can provide enhanced performance to the matrix.

The functional food composition can comprise edible particles for weight loss and/or additional edible particles can have a size with a volume mean distribution between about 0.1 microns and about 1.0 microns, between about 0.1 microns and about 10.0 microns, between about 0.1 microns and about 100.0 microns, between about 0.1 microns and about 1.0 millimeters, or between about 0.1 and about 3 millimeters.

Described herein is a method of preparing a functional food composition, comprising the steps of providing an edible or potable substance, an edible unpolymerized matrix, and edible particles for weight loss, combining the edible or potable substance and/or the edible unpolymerized matrix with the edible particles for weight loss, and encapsulating the edible substance with the edible matrix. The method can further comprise the step of polymerizing the edible matrix.

The functional food composition can be used for weight loss management, for the maintenance of a normal healthy body mass index, for the treatment and prevention of being overweight, and for the treatment and prevention of obesity.

Described is a method for managing weight by administering to an individual in need thereof a functional food composition as described herein. The functional food composition can be administered in an amount sufficient for the maintenance of a normal body mass index. The functional food composition can be administered in an amount sufficient to decrease body mass index.

Described is a method for the treatment of obese body mass index by administering to an individual in need thereof a functional food composition as described herein.

Described is a method for the treatment of overweight body mass index by administering to an individual in need thereof a functional food composition as described herein.

This disclosure also relates to the delivery of compounds useful for skeletal health using edible or potable substances encapsulated in an edible membrane matrix. In particular aspects, the functional food provides functionally nutritive benefits for disease prevention and health wellness through the incorporation of certain biologically active compounds in the edible substance and/or as particles in the membrane matrix.

Described herein is a functional food composition and method for making, comprising an edible or potable substance, a cross-linked matrix encapsulating the edible or potable substance, and a compound that promotes skeletal health. The functional food composition can comprise a compound that promotes (e.g., in the form of edible particles) skeletal health selected from the group consisting of a calcium compound, a magnesium compound, vitamin K₂, a calcium uptake enhancer, and derivatives thereof. The calcium uptake enhancer can be selected from the group consisting of colecalciferol and ergocalciferol. The calcium uptake enhancer can be vitamin D. The vitamin D concentration can be from about 1000 to about 4000 IU's, the vitamin K₂ concentration from about 50 micrograms to about 500 micrograms, the magnesium concentration from about 40 to about 320 milligrams, and the calcium concentration from about 500-2000 milligrams, in an individual functional food composition serving.

The functional food composition can comprise additional edible particles in the cross-linked matrix. The edible particles for skeletal health and/or additional edible particles can provide enhanced performance to the matrix.

The functional food composition edible particles for skeletal health and/or additional edible particles can have a size with a volume mean distribution between about 0.1 microns and about 1.0 microns, between about 0.1 microns and about 10.0 microns, between about 0.1 microns and about 100.0 microns, between about 0.1 microns and about 1.0 millimeters, or between about 0.1 and about 3 millimeters.

Described herein is a method of preparing a functional food composition, comprising the steps of providing an edible or potable substance, an edible unpolymerized matrix, and a compound that promotes skeletal health, combining the edible or potable substance and/or the edible unpolymerized matrix with the compound that promotes skeletal health, and encapsulating the edible substance with the edible matrix. The method can further comprise the step of polymerizing the edible matrix.

The functional food composition can be used for maintenance of skeletal health, the prevention of rickets, the prevention of osteoporosis, the prevention of osteomalacia, to increase bone density, and for the prevention and treatment of cardiovascular disease.

Described is a method for maintaining or improving skeletal health by administering to an individual in need thereof a functional food composition as described herein. The functional food composition can be administered in an amount sufficient to increase bone density in an individual. The functional food composition can be administered in an amount sufficient to decrease chronic joint and skeletal pain.

Described is a method for the prevention and treatment of a skeletal disease by administering to an individual in need thereof a functional food composition as described herein. The skeletal disease can consist of at least one of rickets, osteoporosis, and osteomalacia.

This disclosure also relates to the delivery of bioflavonoids, carotenes and essential fatty acid compounds using edible or potable substances encapsulated in an edible membrane matrix. In particular aspects, the functional food provides functionally nutritive benefits for cardiovascular disease prevention and health wellness through the incorporation of certain biologically active compounds in the edible substance and/or as particles in the membrane matrix.

Described herein is a functional food composition and method for making, comprising an edible or potable substance, a cross-linked matrix encapsulating the edible or potable substance, and a cardiovascular health compound comprising a bioflavonoid, a carotene, and an essential fatty acid.

The functional food composition can include a bioflavonoid derived from at least one of the group consisting of *Camellia sinensis, Theobroma cacao,* varieties thereof, strains thereof, cultivars thereof, and combinations thereof.

The functional food composition can include bioflavonoid selected from the group consisting of esveratrol, quercetin, rutin, catechin, epicatechin, proanthocyanidin, cocoaflavanol, and combinations thereof.

The functional food composition can comprise an essential fatty acid selected from the group consisting of α-linolenic acid, eicosapentanoic acid, docosahexaenoic acid, and combinations thereof.

The functional food composition can comprise carotene selected from the group consisting of α-carotene, β-carotene, γ-carotene, δ-carotene, ε-carotene, lycopene, and combinations thereof

The functional food composition can comprise additional edible particles in the cross-linked matrix. The cardiovascular health compound and/or additional edible particles can provide enhanced performance to the matrix.

The functional food composition of the cardiovascular health compound and/or additional edible particles can have a size with a volume mean distribution between about 0.1 microns and about 1.0 microns, between about 0.1 microns and about 10.0 microns, between about 0.1 microns and about 100.0 microns, between about 0.1 microns and about 1.0 millimeters, or between about 0.1 and about 3 millimeters.

Provided herein is a method of preparing a functional food composition, comprising the steps of providing an edible or potable substance, an edible matrix, and a cardiovascular health compound comprising a bioflavonoid, a carotene, and an essential fatty acid, combining the edible or potable substance and/or the edible matrix with the cardiovascular health compound, and encapsulating the edible substance with the edible matrix. The method can further comprise the step of polymerizing the edible matrix.

The functional food composition can be used for the maintenance of cardiovascular health, for treatment of cardiovascular disease, for treatment of high blood pressure, and for treatment of high blood cholesterol.

Described is a method for the maintenance of cardiovascular health by administering to an individual in need thereof a functional food composition as described herein.

Described is a method for the treatment of cardiovascular disease by administering to an individual in need thereof a functional food composition as described herein.

Described is a method for the treatment of high blood pressure by administering to an individual in need thereof a functional food composition as described herein.

Described is a method for the treatment of high blood cholesterol by administering to an individual in need thereof a functional food composition as described herein.

This disclosure also relates to the delivery of compounds useful for joint and cartilage health using edible or potable substances encapsulated in an edible membrane matrix. In particular aspects, the functional food provides functionally nutritive benefits for disease prevention and health wellness through the incorporation of certain biologically active compounds in the edible substance and/or as particles in the membrane matrix.

Described herein is a functional food composition and method for making, comprising an edible or potable substance, a cross-linked matrix encapsulating the edible or potable substance, and particles for joint and cartilage health. The joint and cartilage health compound may be glucosamine, chondroitin, collagen, hyaluronic acid, salts thereof, complexes thereof, bioavailable derivatives thereof, and bioactive derivatives thereof.

The functional food composition can consist of about 500 milligrams to about 2000 milligrams chondroitin, about 500 milligrams to about 2000 milligrams of glucosamine, about 20 to about 200 milligrams hyaluronic acid, or about 500 milligrams to about 3000 milligrams of collagen.

The functional food composition can include at least one ingredient selected from the group consisting of a vitamin, a mineral, an amino acid, an antioxidant, an anti-inflammatory agent, and an essential fatty acid.

The functional food composition can comprise additional edible particles in the cross-linked matrix. The edible particles for joint and cartilage health and/or additional edible particles can provide enhanced performance to the matrix.

The functional food composition can comprise edible particles for joint and cartilage health and/or additional edible particles can have a size with a volume mean distribution between about 0.1 microns and about 1.0 microns, between about 0.1 microns and about 10.0 microns, between about 0.1 microns and about 100.0 microns, between about 0.1 microns and about 1.0 millimeters, or between about 0.1 and about 3 millimeters.

Described herein is a method of preparing a functional food composition, comprising the steps of providing an edible or potable substance, an edible unpolymerized matrix, and edible particles for joint and cartilage health, combining the edible or potable substance and/or the edible unpolymerized matrix with the edible particles for joint and cartilage health, and encapsulating the edible substance with the edible matrix. The method can further comprise the step of polymerizing the edible matrix.

The functional food composition can be used for maintenance of joint and cartilage health, the treatment and prevention of arthritis, the treatment and prevention of osteoarthritis, the treatment and prevention of rheumatoid arthritis, the treatment and prevention of psoriatic arthritis, the treatment and prevention of joint effusion, the treatment and prevention of joint pain, the treatment and prevention of joint inflammation, the treatment and prevention of synovitis, and for post-operative care.

Described is a method for maintaining or improving joint and cartilage health by administering to an individual in need thereof a functional food composition as described herein. The functional food composition can be administered in an amount sufficient to decrease chronic joint pain in an individual.

Described is a method for the prevention and treatment of a degenerative joint disease by administering to an individual in need thereof a functional food composition as described herein. The degenerative joint disease can consist of at least one of the group consisting of arthritis, joint effusion, and deterioration of proper joint function, and the arthritis can consist of at least one of the group selected from osteoarthritis, rheumatoid arthritis, and psoriatic arthritis.

Described is a method for the prevention and treatment of a joint disorder by administering to an individual in need thereof a functional food composition as described herein. The joint disorder can consist of at least one of the group selected from joint inflammation, chronic joint pain, and synovitis.

This disclosure also relates to the delivery of multivitamin compounds using edible or potable substances encapsulated in an edible membrane matrix. In particular aspects, the functional food provides functionally nutritive benefits for disease prevention and health wellness through the incorporation of certain biologically active compounds in the edible substance and/or as particles in the membrane matrix.

Described herein is a functional food composition and method for making, comprising an edible or potable substance, a cross-linked matrix encapsulating the edible or potable substance, and a multivitamin compound. The functional food composition can include a multivitamin wherein the multivitamin compound is at least two of the group consisting of vitamin A, vitamin B₁, vitamin B₂, vitamin B₃, vitamin B₅, vitamin B₆, vitamin B₉, vitamin B₁₂, vitamin C, vitamin D₃, vitamin E, vitamin K₁, vitamin K₂, vitamin H (biotin), and combinations thereof.

The functional food composition can be a multivitamin compound further comprising minerals. The functional food composition mineral can be at least one of the group selected from boron, calcium, chloride, chromium, cobalt, copper, fluorine, iodine, iron, magnesium, manganese, molybdenum, nickel, phosphorous, potassium, selenium, silicon, sodium, strontium, sulfur, vanadium, zinc, and combinations thereof.

The functional food composition can further comprise at least one of the group consisting of an amino acid, an antioxidant, a carotenoid, a flavonoid, an isoflavone, a nutraceutical, and combinations thereof.

The functional food composition can comprise additional edible particles in the cross-linked matrix. The multivitamin compound and/or additional edible particles can provide enhanced performance to the matrix.

The functional food composition edible multivitamin compound and/or additional edible particles can have a size with a volume mean distribution between about 0.1 microns and about 1.0 microns, between about 0.1 microns and about 10.0 microns, between about 0.1 microns and about 100.0 microns, between about 0.1 microns and about 1.0 millimeters, or between about 0.1 and about 3 millimeters.

Described herein is a method of preparing a functional food composition, comprising the steps of providing an edible or potable substance, an edible matrix, and a multivitamin compound, combining the edible or potable substance and/or the edible matrix with the multivitamin compound, and encapsulating the edible substance with the edible matrix. The method can further comprise the step of polymerizing the edible matrix.

The functional food composition can be used for maintenance of recommended daily allowance of vitamins and minerals, for the treatment of vitamin and mineral deficiency related to dietary imbalance, for the treatment of vitamin and mineral deficiency related to chronic disease, for the treatment of vitamin and mineral deficiency related to the treatment of a chronic disease, for the treatment of vitamin and mineral deficiency related to a weight loss diet.

Described is a method for the treatment of vitamin and mineral deficiency by administering to an individual in need thereof a functional food composition as described herein.

Described is a method for the maintenance of recommended daily allowances of vitamins and minerals by administering to an individual in need thereof a functional food composition as described herein.

This disclosure also relates to the delivery of essential fatty acid compounds using edible or potable substances encapsulated in an edible membrane matrix. In particular aspects, the functional food provides functionally nutritive benefits for disease prevention and health wellness through the incorporation of certain biologically active compounds in the edible substance and/or as particles in the membrane matrix.

Described herein is a functional food composition and method for making, comprising an edible or potable substance, a cross-linked matrix encapsulating the edible or potable substance, and an essential fatty acid compound. The functional food composition can include an essential fatty acid compound that is an essential fatty acid. The functional food composition essential fatty acid can be selected from the group consisting of α-linolenic acid, eicosapentanoic acid, docosahexaenoic acid, and combinations thereof.

The functional food composition can further comprise dietary fiber. The dietary fiber can be selected from the group consisting of galacto-oligosaccharides, inulin and oligofructose (fructo-oligosaccharide), isomalto-oligosaccharides, lactulose, lactosucrose, transgalacto-oligosaccharides, soybean oligosaccharides, tagatose, xylo-oligosaccharides, and combinations thereof.

The functional food composition can comprise additional edible particles in the cross-linked matrix. The essential fatty acid compound, dietary fiber, and/or additional edible particles can provide enhanced performance to the matrix.

The functional food composition essential fatty acids, dietary fiber, and/or additional edible particles can have a size with a volume mean distribution between about 0.1 microns and about 1.0 microns, between about 0.1 microns and about 10.0 microns, between about 0.1 microns and about 100.0 microns, between about 0.1 microns and about 1.0 millimeters, or between about 0.1 and about 3 millimeters.

Described herein is a method of preparing a functional food composition, comprising the steps of providing an edible or potable substance, an edible unpolymerized matrix, and an essential fatty acid compound, combining the edible or potable substance and/or the edible unpolymerized matrix with the essential fatty acid compound, and encapsulating the edible substance with the edible matrix. The method can further comprise the step of polymerizing the edible matrix.

The functional food composition can be used for promoting normal development of the brain, eyes and nerves, for maintenance of normal development of the brain, eyes and nerves, for maintenance of brain, eye and nerve health, for the prevention and treatment of coronary disease, for the prevention and treatment of dementia, for the prevention and treatment of depression, and for the prevention and treatment of arthritis.

Described is a method for promoting normal development and health maintenance of the brain, eyes and nerves by administering to an individual in need thereof a functional food composition as described herein.

Described is a method for the treatment of coronary disease by administering to an individual in need thereof a functional food composition as described herein.

Described is a method for the maintenance of coronary health by administering to an individual in need thereof a functional food composition as described herein.

Described herein is a method for the prevention and treatment of dementia by administering to an individual in need thereof a functional food composition as described herein.

Described is a method for the prevention and treatment of depression by administering to an individual in need thereof a functional food composition as described herein.

Described is a method for the prevention and treatment of arthritis by administering to an individual in need thereof a functional food composition as described herein.

This disclosure also relates to the delivery of branched chain amino acids using edible or potable substances encapsulated in an edible membrane matrix. In particular aspects, the functional food provides functionally nutritive benefits for consumers by contributing to the treatment and prevention of certain diseases and disorders and the maintenance of general health and wellness through the incorporation of certain biologically active compounds in the edible substance and/or as particles in the membrane matrix.

Described herein is a functional food composition and method for making, comprising an edible or potable substance, a cross-linked matrix encapsulating the edible or potable substance, the cross-linked matrix comprising an edible polymer, and a branched chain amino acid.

The functional food composition can further comprise L-glutamine.

The functional food composition branched chain amino acid can be selected from the group consisting of L-leucine, L-isoleucine, L-valine, and combinations thereof.

The functional food composition can further comprise a multivitamin composition.

The functional food composition can comprise additional edible particles in the cross-linked matrix. The branched chain amino acid, L-glutamine, and/or additional edible particles can provide enhanced performance to the matrix.

The functional food composition of the branched chain amino acid, L-glutamine, and/or additional edible particles can have a size with a volume mean distribution between about 0.1 microns and about 1.0 microns, between about 0.1 microns and about 10.0 microns, between about 0.1 microns and about 100.0 microns, between about 0.1 microns and about 1.0 millimeters, or between about 0.1 and about 3 millimeters.

Described herein is a method of preparing a functional food composition, comprising the steps of providing an edible or potable substance, an edible matrix, and a branched chain amino acid, combining the edible or potable substance and/or the edible matrix with the branched chain amino acid, and encapsulating the edible substance with the edible matrix. The method can further comprise the step of polymerizing the edible matrix.

The functional food composition can be used for the maintenance of muscle mass, to increase muscle mass, for the prevention and treatment of muscle mass loss during post-operative care, for the prevention and treatment of muscle mass loss during trauma care, for the prevention and treatment of muscle mass loss from malignant disease, and for the prevention and treatment of muscle mass loss from burn care.

Described is method for the maintenance of muscle mass by administering to an individual in need thereof a functional food composition as described herein.

Described is a method to increase muscle mass by administering to an individual in need thereof a functional food composition as described herein.

Described is a method for the prevention and treatment of muscle mass loss during post-operative care by administering to an individual in need thereof a functional food composition as described herein.

Described is a method for the prevention and treatment of muscle mass loss during trauma care by administering to an individual in need thereof a functional food composition as described herein.

Described is a method for the prevention and treatment of muscle mass loss from malignant disease by administering to an individual in need thereof a functional food composition as described herein.

Described is a method for the prevention and treatment of muscle mass loss from burn care by administering to an individual in need thereof a functional food composition as described herein.

This disclosure also relates to the delivery of antioxidants using edible or potable substances encapsulated in an edible membrane matrix. In particular aspects, the functional food provides functionally nutritive benefits for consumers by contributing to the treatment and prevention of certain diseases and disorders and the maintenance of general health and wellness through the incorporation of certain biologically active compounds in the edible substance and/or as particles in the membrane matrix.

Described herein is a functional food composition and method for making, comprising an edible or potable substance, a cross-linked matrix encapsulating the edible or potable substance, the cross-linked matrix comprising an edible polymer and an edible antioxidant containing compound characterized by oxygen radical absorbance capacity above about 1000 per 100 grams.

The functional food composition can include edible particles for at least one of the group consisting of a bioflavonoid, a carotene, a vitamin, and combinations thereof, wherein the bioflavonoid is selected from the group consisting of esveratrol, quercetin, rutin, catechin, epicatechin, proanthocyanidin, and combinations thereof, wherein the carotene is selected from the group consisting of α-carotene, β-carotene, γ-carotene, δ-carotene, ε-carotene, lycopene, and combinations thereof, and wherein the vitamin is selected from the group consisting of vitamin A, vitamin C, vitamin E, and combinations thereof.

The functional food composition can further comprise a dietary fiber.

The functional food composition can comprise additional edible particles in the cross-linked matrix. The edible antioxidant containing compound compound and/or additional edible particles can provide enhanced performance to the matrix.

The functional food composition cross-linked matrix can have a thickness from about 10 microns to about 200 millimeters.

The functional food composition of the edible antioxidant containing compound and/or additional edible particles can have a size with a volume mean distribution between about 0.1 microns and about 1.0 microns, between about 0.1 microns and about 10.0 microns, between about 0.1 microns and about 100.0 microns, between about 0.1 microns and about 1.0 millimeters, or between about 0.1 and about 3 millimeters.

Described herein is a method of preparing a functional food composition, comprising the steps of providing an edible or potable substance, an edible unpolymerized matrix, and particles of an edible antioxidant compound, wherein the edible antioxidant containing compound is characterized by an oxygen radical absorbance capacity above about 1000 per 100 grams, combining the edible or potable substance and/or the edible unpolymerized matrix with the edible antioxidant containing compound, and encapsulating the edible substance with the edible matrix. The method can further comprise the step of polymerizing the edible matrix.

The functional food composition can be used for the maintenance of skin health, for the maintenance of nails, for the maintenance of hair, for the prevention and treatment of dry skin, for the prevention and treatment of skin wrinkles, for the prevention and treatment of skin spots, for the prevention and treatment of brittle nails, for the prevention and treatment of hair loss, for the maintenance of cardiovascular health, for the treatment of cardiovascular disease, for the maintenance of brain function and mental acuity, for the maintenance of vision health, for the maintenance of healthy blood sugar levels, and for the maintenance of urinary tract health.

Described is a method for the maintenance of cardiovascular health by administering to an individual in need thereof a functional food composition as described herein.

Described is a method for the treatment of cardiovascular disease by administering to an individual in need thereof a functional food composition as described herein.

Described is a method for the maintenance of skin health by administering to an individual in need thereof a functional food composition as described herein.

Described is a method for the maintenance of hair health by administering to an individual in need thereof a functional food composition as described herein.

Described is a method for the maintenance of nail health by administering to an individual in need thereof a functional food composition as described herein.

Described is a method for the prevention and treatment of hair loss by administering to an individual in need thereof a functional food composition as described herein.

Described is a method for the prevention and treatment of brittle nails by administering to an individual in need thereof a functional food composition as described herein.

Described is a method for the prevention and treatment of skin wrinkles by administering to an individual in need thereof a functional food composition as described herein.

Described is a method for the prevention and treatment of skin spots by administering to an individual in need thereof a functional food composition as described herein.

Described is a method for the prevention and treatment of high blood cholesterol by administering to an individual in need thereof a functional food composition as described herein.

Described is a method for the maintenance of brain function and mental acuity by administering to an individual in need thereof a functional food composition as described herein.

Described is a method for the maintenance of vision health by administering to an individual in need thereof a functional food composition as described herein.

Described is a method for the maintenance of healthy blood sugar levels by administering to an individual in need thereof a functional food composition as described herein.

Described is a method for the maintenance of urinary tract health by administering to an individual in need thereof a functional food composition as described herein.

This disclosure also relates to functional food compositions. Functional food compositions are designed to deliver sufficient quantities and quality of β-glucans and probiotics using edible or potable substances encapsulated in an edible membrane matrix. In particular aspects, the functional food composition provides nutritive and health benefits for immunological response, disease treatment and prevention, and health maintenance through the incorporation of certain biologically active compounds in the edible substance and/or as particles in the membrane matrix.

Described herein is a functional food composition and methods for making, comprising an edible or potable substance, a cross-linked matrix encapsulating the edible or potable substance, a probiotic, and β-glucan. The matrix can comprise the β-glucan and the edible or potable substance component comprises the probiotic, or the matrix comprises the probiotic and the edible or potable substance comprises the β-glucan. The matrix can comprise the probiotic and the β-glucan, or the edible or potable substance can comprise the probiotic and the β-glucan.

The functional food composition can comprise a probiotic concentration from about 1.0 to about 100 billion colony forming units in an individual edible transport vessel, and the probiotic can be an organism selected from the genera group consisting of *Aspergillus, Bacillus, Bacteroides, Bifidobacterium, Brettanomyces, Enterococcum, Kluyveromyces, Lactobacillus, Lactococcus, Leuconostoc, Pediococcus, Propionibacterium, Saccharomyces, Shewanella, Streptococcus, Torulaspora, Vagococcus,* and any derivatives, strains, and combinations thereof.

The functional food composition can comprise a probiotic that is heat killed. The heat killed probiotic can be in the functional food in amounts of about 100 mg to about 500 mg.

The functional food composition can comprise a β-glucan where the β-glucan is (1,3)-(1,6)-β-glucan. The β-glucan can be (1,3)-β-glycosidic linked D-glucose subunits β -(1,6)-linked to (1,3)-β-glycosidic linked D-glucose side chains of varying length and bonded to the backbone subunits at irregular or regular intervals. The β-glucan concentration can be from about 1.0 milligrams to 5.0 grams.

The functional food composition can comprise additional edible particles in the cross-linked matrix. The β-glucan, the probiotic, and/or additional edible particles can provide enhanced performance to the matrix.

The β-glucan, the probiotic, and/or additional edible particles can comprise a size having a volume mean distribution between about 0.1 microns and about 1.0 microns, between about 0.1 microns and about 10.0 microns, between about 0.1 microns and about 100.0 microns, between about 0.1 microns and about 1.0 millimeters, or between about 0.1 and about 3 millimeters.

The functional food composition can be used for increasing beneficial immune responses, for decreasing adverse immune responses, for maintaining healthy immune responses, for enhancement of a vaccination response, to stimulate antibody production, to reduce the occurrence of symptomatic common cold infections, for treating fungal infections, for treating *Candida albicans* infections, for a free-radical scavenger, for treatment of cancer, and for protection against radiation exposure.

Described is a method for maintaining or improving immune health by administering to an individual in need thereof a functional food composition as described herein. The functional food composition can be administered in an amount sufficient to stimulate antibody production, for enhancement of a vaccination response, to reduce the occurrence of symptomatic common cold infections, for treating fungal infections, for treating *Candida albicans* infections, for use as a free-radical scavenger, for treatment of cancer, for protection against radiation.

In all functional food compositions, the matrix can be comprised of an edible polymer charge cross-linked by multivalent ions, including cross-linking interactions between the edible particles and edible polymer or plurality of edible polymers via bridges formed by the multivalent ions. The matrix can comprise a polysaccharide selected from the group consisting of a hydrocolloid, shellac, and fibers.

The cross-linked matrix of the functional food composition can have a thickness from about 10 microns to about 200 millimeters.

The functional food composition can have an edible or potable substance comprising at least one of a powder, a gel, an emulsion, a foam, a solid, and combinations thereof. The edible or potable substance can be selected from the group consisting of fruit, vegetable, meat, a dairy product, a carbohydrate food product, a botanical, a confection, and combinations thereof.

The functional food composition can have an edible or potable substance comprising a liquid, particularly wherein the liquid comprises at least one of water, an alcohol, a juice, an alcohol mixed drink, a coffee product, a tea product, a soft drink, an energy supplement product, a dietary supplement, a confection, fortified substances thereof, and combinations thereof.

The functional food composition can have an edible or potable substance defining a volume of between about 5 and 1,250 cubic centimeters, and the cross-linked matrix has a strength sufficient to contain and transport a liquid water volume equivalent to the interior volume defined by the edible or potable substance.

### DESCRIPTION OF DRAWINGS

Figure 1 shows the chemical structure of an alginate polymer -(M)ₘ-(G)ₙ- (M: mannuronate; G: guluronate).
Figure 2 illustrates polymerization of sodium alginates via divalent cations (e.g., Ca²⁺).
Figure 3 is a schematic illustrating bonding between positive particles (e.g., Ca²⁺ or Mg²⁺) and negative particles (e.g., alginate or food particles).
Figure 4 illustrates multiple transport systems arranged in shells.
Figure 5 illustrates a transport system having large particles suspended in an outer membrane layer.
Figure 6 illustrates a transport system having small particles suspended in an outer membrane layer.
Figure 7 illustrates a transport system having both large and small particles suspended in an outer membrane layer.
Figure 8 illustrates a transport system having an outer membrane layer that is non-uniformly shaped.
Figures 9A-9C illustrate a multimembrane composition with particles embedded in the membrane and a particulate layer between membranes.

### DETAILED DESCRIPTION

### Definitions

**B-glucan** refers generally to chains of D-glucose polysaccharides linked by beta-type glycosidic bonds.

**(β-1,3):(β-1,6) D-glucan** and **(1,3)-(1,6)-β-glucan** refer to glucose polysaccharides consisting of a backbone of (1,3)-β-glycosidic linked D-glucose subunits β -(1,6)-linked to (1,3)-β-glycosidic linked D-glucose side chains of varying length and bonded to the backbone subunits at irregular or regular intervals.

**"Bioavailable"** and **"bioavailability"** refer to the quantity, fraction or proportion of the administered or ingested substance capable of being absorbed and available for use or storage, and varies according to physiological state and nutritional status of a consumer.

**"Bioflavonoid"** refers to flavones, isoflavanoids and neoflavanoids, subclasses, sub-groups, and chemically related compounds thereof, including flavanols such as catechins and epicatechins, as used and defined according to IUPAC nomenclature and designation.

**"Biologically active compound"** and **"biologically active agent"** refer to any compound that elicits a response in or from a biological system and/or organism.

**"Body Mass Index"** refers to a measure of body fat based on height and weight that applies to adult men and women, wherein the quantitative measure is used to categorize an adult's weight as underweight, normal weight, overweight, and obese.

**"Branched chain amino acid"** refers to the essential amino acids L-leucine, L-isoleucine, and L-valine, separately or in combination.

**"Elastic tissue component"** refers to protein compounds derived from or located in skin, bone, connective tissue such as cartilage, tendons and ligaments, and/or other fibrous tissues as found in vertebrates, including, without limitation, collagen and elastin, that are characterized by increased tensile strength and/or elasticity.

**"Essential fatty acid"** refers to omega-3 fatty acids that cannot be synthesized *de novo* by the human body, and includes α-linoleic acid (ALA), eicosapentaenoic acid (EPA), and docosahexaenoic acid (DHA). It is known that diets including the shorter-chained omega-3 fatty acid ALA, will allow for the human body to form long-chain omega-3 fatty acid EPA and then from EPA, the most crucial, DHA, but this process has low efficiency.

**"Fortified"** or **"fortify"** refers to the adding of ingredients to something or to add further ingredients to food or drink in order to improve its flavor or add nutrients.

**"Functional food"** refers to an edible composition fortified with known biologically active compounds or agents that provide enhanced benefit (e.g. a health benefit, nutritional benefit) to the consumer. The enhanced benefit can be quantitative, qualitative or subjective and include for example, health promotion and disease prevention. In general, the functional food is fortified with defined (qualitative or quantitative) amounts of the known beneficial ingredients to provide the desired benefit.

**"Integument"** refers to skin and protein based skin cell products thereof, including hair and finger/toe nails.

**"Matrix"** refers to the molecular three dimensional architecture of a polymerized or unpolymerized polymer or copolymeric substance.

**"Matrices"** refers to two or more of a distinct matrix.

**"Membrane"** as used herein refers to the polymerized polymers and/or co-polymers encapsulating an edible or potable substance.

**"Microbiota"** refers to the collective term for microflora (i.e., any type of minute organism) that may be found within a given environment.

**"Microflora"** refers to living microscopic microorganisms that maintain a more or less constant presence in a particular area of living biological system, and includes bacteria, viruses, protozoa, yeasts and fungi.

**"Nutraceutical"** refers to a product isolated or purified, often from foods, that is generally sold in medicinal forms not usually associated with food, includes dietary supplements and food-related products for promoting health.

**"ORAC"** refers to a method of determining **o**xygen **r**adical **a**bsorbance capacity. Data from the ORAC method shows antioxidant capacity of biologically active compounds *in vitro.*

**"Probiotic"** refers to living microorganisms that, on ingestion by a host organism, and in sufficient concentration, exert health benefits to the host organism beyond basic nutrition.

**"Prebiotic"** refers to a selectively fermented ingredient that allows specific changes, both in the composition and/or activity in the gastrointestinal microbiota that confers benefits upon host well-being and health.

**"Shells"** refers to relatively unpliable to hard polymerized polymers or co-polymers.

**"Skins"** as used herein refers to the polymerized polymers and/or co-polymers encapsulating an edible or potable substance.

### I. Overview of the prebiotic/probiotic functional food composition

The disclosure relates to functional food compositions that contain a prebiotic and a probiotic when, upon ingestion, promote gastrointestinal health and well-being. The functional food composition contains an edible or potable substance that is encapsulated in an edible membrane, and further contains a probiotic and a prebiotic. The functional food compositions described herein provide a convenient and improved way for individuals to consume probiotics in quantities needed to promote gastrointestinal health and well-being.

Generally, functional food compositions are intended to provide a healthy benefit to a consumer. The benefit can be qualitative, quantitative or subjective and include, for example, health promotion, disease prevention, etc. Some benefits include providing an important nutritive food source, vitamins and supplements, or probiotics, which contributes to the prevention, management and treatment of chronic diseases or disorders. This health improvement benefit can also be provided to a consumer when additional ingredients or more of an existing ingredient are added to a food so that the benefit from the food product is gained. However, delivery systems for some bioavailable compounds including prebiotics and probiotics often reduce their viability, resulting in suboptimal quantities of these compounds being made available to the consumer.

The functional food compositions described herein provide for a significant improvement in the viability and concentration of probiotics available to a consumer. Within these compositions, the prebiotic compounds and probiotic compounds are compartmentalized so that the edible or potable substance and the encapsulating membrane do not both contain the probiotic or both contain the prebiotic. Upon mastication and ingestion, the probiotic and the prebiotic are released from the functional food composition, and combined in the intestinal tract wherein the prebiotic provides an immediately available nutrition source for the probiotic to grow. Prebiotics and probiotics can thus be engineered and matched to each other to provide an optimal condition for quick population in the gut upon consumption by a host.

The functional food compositions described herein are designed to provide prebiotics and probiotics in biologically effective quantities superior to commonly used products. The compositions are particularly well-suited for providing a larger active ingredient load as well as being amenable for controlling the actual portion size for an intended health benefit. An eight ounce serving of commercial yogurt in plastic containers commonly provides a microbial load of less than 1 billion colony forming units (BCFU), far below a bacterial load level sufficient to provide health benefits. The compositions described herein can be designed to provide up to 100 BCFU's.

These functional food compositions also enhance the stability, gustatory experience, and delivery of functional biologically active compounds. Functional food compositions contain and protect ingestible/edible substances, such as ice cream, yogurts, etc. and probiotics or prebiotics, by encapsulating the substance within edible or biodegradable membranes. The edible membranes of these compositions can be formed from various natural polymeric substances allowing different compositions to be easily transported and consumed.

Considerations for the choice of probiotic or probiotic combinations for use in the function food compositions are that the probiotic remain viable until ingestion in a biologically effective concentration, and the probiotic be able to confer a health benefit to and/or use for treatment of a gastro-intestinal, immune or other systemic disorder in the host organism. In all instances, it is desirable that these food compositions (product) include effectual probiotics to re-establish healthy intestinal bacterial flora or otherwise confer a health benefit to a host organism ingesting the probiotic.

Important variables when specifically considering a prebiotic and a probiotic for use in the functional food composition include: 1) the interaction between the prebiotic and probiotic, 2) the by-product left by the probiotic, 3) the taste and/or texture of the edible composition after the prebiotic and probiotic interact, 4) the rate of replication of the probiotic, and 5) the health benefit(s) the probiotic and/or prebiotic may convey.

In general, the prebiotic and the probiotic are substantially separated from each other so that the matrix component and the edible or potable substance component contain the prebiotic or the probiotic. If the matrix component includes the prebiotic, the edible or potable substance component can include the probiotic. If the matrix component includes the probiotic, the edible or potable substance component can include the prebiotic. In some applications, the location of the prebiotic and the probiotic may be conditionally dependent on each other. The effectiveness of the prebiotic or probiotic may be optimized based on placement within either the matrix or the edible or potable composition or they may be equally as effective in either location.

In general, the prebiotic and probiotic are separated in the composition so there is no interaction between the two compounds, and are designed to be released from the food product after or upon consumption. After consuming the serving of functional food composition, the prebiotic may be broken down by the body's natural microflora which could provide a better suited environment for the additional probiotics delivered by the edible product. The prebiotic and probiotic may interact after the edible composition is broken down and before the edible composition reaches the gastrointestinal tract (*e.g*., chewing, etc.). Mastication and ingestion release the components from the membrane matrix and the edible or potable substance to allow them mix, interact to allow for growth, and to colonize in the gut in sufficient amounts.

If desired, the functional food compositions can be prepared so that the prebiotic and probiotic are allowed to interact within the food product by diffusion from the membrane to the encapsulated food or vice versa. In these instances, the physical and/or chemical characteristics of the membrane can change as the prebiotic is used by the probiotic. As the prebiotic is used by the probiotic over time, the membrane can be altered in terms of its physical and/or chemical characteristics, for example, permeability, elasticity, color, taste, tensile strength, texture, pH, overall charge, etc. The changes occurring with the edible composition can be an indicator (e.g., the matrix displays a noticeable color change to indicate a sufficient number of probiotics has been reached, etc.). In some embodiments, the consumption of the prebiotic by the probiotic leaves behind by-products that affect the mouth feel or user experience of the edible composition (e.g., carbonation, etc.). In some embodiments, the by-products left in the edible product by the probiotic confer separate health benefits and/or alter the physical and/or chemical characteristics of the edible composition.

In some applications, the matrix pre- or probiotic component may diffuse into the edible or potable product while the edible or potable substance pre- or probiotic component remains in the edible or potable substance. Similarly, the edible or potable substance pre- or probiotic component can diffuse into the matrix while the matrix pre- or probiotic component remains in the matrix. After diffusion, the matrix pre- or probiotic component and the edible or potable substance pre- or probiotic component may interact. The edible matrix also can affect the diffusion rate between the probiotic microorganism and the prebiotic nutrient source for the microorganism based on type of material, charge, subunit composition, and/or embedded particles. If the probiotic diffuses out of the membrane, it may implement its valuable properties on the surrounding environment (e.g., the digestive tract, etc.). The matrix can be selectively permeable for prebiotic and/or probiotic diffusion while the edible product remains encapsulated and substances outside of the embodiment are repelled.

It may be desirable that the prebiotic and probiotic remain in their respective locations within the edible composition, but the probiotic may access the embedded prebiotic at the matrix-edible or potable substance interface and the probiotic may flourish within its respective location. The probiotic may use the prebiotic entirely, partially, or not at all while embodied within the same edible composition. If the prebiotic is in the edible composition in excess for the probiotic, the prebiotic may act as nutritional support for both the probiotic and the consumer.

Health benefit aspects contemplated for use of the pre- and probiotic functional food compositions described herein include relief from intestinal inflammation, pathogen related diarrhea, antibiotic related diarrhea, various irritable bowel syndromes, urogenital infections, allergies, lactose intolerance, colon cancer, high cholesterol, high blood pressure, immunity function, immune based infections, peptic ulcers, inflammation, colitis, necrotizing enterocolitis, Crohn's Disease, eczema, Inflammatory Bowel Disease, vitamin production, etc. Health and wellness preventative treatment with probiotics includes decreased intestinal gas and bloating, increased fecal transit time, relief from constipation, improved digestion, increased flora count in stool, etc. For example, probiotics may beneficially affect the host by augmenting the host intestinal microbial population beyond the amount already existing, thus possibly inhibiting unhealthy or harmful pathogens. Furthermore, improvement of these and other gastrointestinal disorders may raise the overall quality of life and daily functional capacity by increasing the regularity of bowel movements and easing pain related to certain disorders.

In general, prebiotics are included in the membrane and/or the edible or potable substance to further promote probiotic activity. The three criteria used for prebiotic classification are resistance to digestion, fermentation availability by intestinal microflora, and the selective stimulation of the growth and/or activity of intestinal bacteria associated with health and wellbeing. The prebiotics described herein are fortified within the functional food composition, in concentration and/or type, to be in addition to natural levels of a prebiotic in the food source.

As described in more detail herein, the common probiotics include lactic acid bacteria, bifidobacteria, yeasts and bacilli, and are often consumed as part of a fermented food source, (e.g., yogurt, soy yogurt, etc.) with added live microbiological cultures.

Measurable outcomes for general health maintenance can be determined quantitatively and/or qualitatively by monitoring, for example, a patient's or consumer's change in composition of intestinal microflora, possibly feelings of well-being, and bowel regularity after a program or regimen of daily consumption of the functional food composition is established. For pretreatment/prevention of diseases, conditions and/or syndroms, the consumer or patient may show milder symptoms or no symptoms. In acute conditions for which the consumer or patient is using the functional food composition, for example diarrheal conditions (i.e. traveler's, antibiotic or viral), the outcome would be faster resolution and reduction in severity for treatment. Chronic conditions such as irritable bowel syndrome, inflammatory bowel disease, Crohn's Disease, etc., a patient or consumer would show measurable reduction in number of episodes and severity of diarrhea and or constipation. In general, a reduction in abdominal pain and discomfort, improvement in quality of life and ability to perform activities of daily living, measurable differences in frequency of episodic conditions and evaluation of changes in concentration of gastrointestinal microflora are all indicators of the health benefits when consuming the functional food compositions for gastrointestinal health described herein .

### Prebiotic and Probiotic compositions

Common probiotics include lactic acid bacteria, bifidobacteria, yeasts and bacilli, and are often consumed as part of a fermented food source, (e.g., yogurt, soy yogurt, etc.) with added live microbiological cultures. Probiotics in concentration and/or type contained within the functional food compositions included herein may be used in addition to natural levels of probiotics in the food source.

Probiotics for use in the foods described herein include, but are not limited to, *Aspergillus niger, Aspergillus oryzae, Bacillus coagulans, Bacillus lentus, Bacillus licheniformis, Bacillus pumilus, Bacillus subtilis, Bacteroides amylophilus, Bacteroides capillosus, Bacteroides ruminocola, Bacteroides suis, Bifidobacterium animalis, Bifidobacterium bifidum, Bifidobacterium breve, Bifidobacterium infantis, Bifidobacterium licheniformis, Bifidobacterium longum, Bifidobacterium pseudo longum, Bifidobacterium subtilus, Bifidobacterium thermophilum, Enterococcus cremoris, Enterococcus diacetylactis, Enterococcus faecium, Enterococcus thermophilus, Lactobacillus acidophilus, Lactobacillus amylovorus, Lactobacillus ansporogenes, Lactobacillus brevis, Lactobacillus buchneri, Lactobacillus bulgaris, Lactobacillus casei, Lactobacillus caucasicus, Lactobacillus cellobiosus, Lactobacillus crispatus, Lactobacillus curvatus, Lactobacillus delbrueckii, Lactobacillus farciminis, Lactobacillus fermentum, Lactobacillus gallinarum, Lactobacillus gasseri, Lactobacillus GG, Lactobacillus helveticus, Lactobacillus johnsonii, Lactobacillus kefir, Lactobacillus lactis, Lactobacillus plantarum, Lactobacillus reuteri, Lactobacillus rhamnosus* (casei), *Lactobacillus salivarius, Lactococcus lactis, Leuconostoc mesenteroides, Pediococcus acidilactici, Pediococcus acidilacticii, Pediococcus cerevisiae, Pediococcus pentosaceus, Propionbacterium freudenreichii, Propionibacterium shermanii, Saccharomyces boulardii, Saccharomyces cerevisiae, Shewanella colwelliana, Shewanella olleyana, Shewanella putrefaciens, Streptococcus cremoris, Streptococcus faecium, Streptococcus infantis, Streptococcus thermophilus, Vagococcus fluvialis, Bacillus coagulans* GBI-30, 6086, *Bifidobacterium animalis* DN 173 010, *Bifidobacterium animalis* subsp. *lactis* BB-12, *Bifidobacterium breve* Yakult, *Bifidobacterium infantis* 35624, *Bifidobacterium lactis* HN019 (DR10), *Bifidobacterium longum* BB536, *Bifidobacterium longum* subsp. *infantis* 35624, *Enterococcus* LAB SF 68, *Escerichia coli* Nissle 1917, *Lactobacillus acidophilus* NCFM, *Lactobacillus acidophilus* LA-5, *Lactobacillus acidophilus* NCFM, *Lactobacillus casei* subspecies *casei, Lactobacillus casei* DN-114 001, *Lactobacillus casei* CRL431, *Lactobacillus casei* F19, *Lactobacillus casei* Shirota, *Lactobacillus delbrueckii* subspecies *bulgaricus, Lactobacillus delbrueckii* subspecies *lactis, Lactobacillus paracasei* Stl 1 (or NCC2461), *Lactobacillus johnsonii* La1 (Lj1), *Lactobacillus johnsonii* NCC533, *Lactobacillus plantarum* 299v, *Lactobacillus rhamnosus* ATCC 53013 (LG), *Lactobacillus rhamnosus* LB21, *Lactobacillus reuteri* ATCC 55730, *Lactobacillus salivarius* (UCC118), *Lactobacillus reuteri* SD2112, *Lactococcus lactis* L1A, *Lactococcus lactis* subspecies *lactis, Lactococcus lactis* subspecies *lactis* biovariant *diacetylactis, Lactococcus lactis* subspecies *cremoris, Leuconostoc mesenteroides* subspecies *cremoris, Leuconostoc mesenteroides* subspecies *dextranicum, Lactobacillus reuteri* Protectis (DSM 17938, daughter strain of ATCC 55730), *Saccharomyces cerevisiae boulardii, Saccharomyces cerevisiae boulardii* (lyo), *Lactobacillus bulgaricus, Streptococcus thermophiles, Bifidobacterium* spp, and any other strains, derivatives, and combinations thereof, including, but not limited to, *Lactobacillus rhamnosus* GR-1 & *Lactobacillus reuteri* RC-14, *Lactobacillus acidophilus* CL 1285 & *Lactobacillus casei* Lbc80r, *Lactobacillus rhamnosus (ATT SD5675)* & *Lactobacillus acidophilus* (STCC SD5221), VSL #3 (a combination of 1 strain of *Streptococcus* thermophiles, four strains of *Lactobacillus* species, & three *Bifidobacterium* species strains from Sigma-Tau Pharmaceuticals), *Lactobacillus acidophilus* NCFM & *Bifidobacterium bifidum* BB-12, *Lactobacillus acidophilus* CUL60 & *Bifidobacterium bifidum* CUL20, *Lactobacillus acidophilus* CL1285 & *Lactobacillus casei* LBC80R, *Lactobacillus helveticus* R0052 & *Lactobacillus rhamnosus* R0011, *Bacillus clausii* (strains O/C, NR, SIN and T), *Lactobacillus plantarum* HEAL 9 & *Lactobacillus paracasei* 8700:2, other genetic variants thereof, and combinations and other combinations thereof.

When the dosage or intake of the effective dose of the active ingredients of the functional food composition are indicated by the number (concentration) of the probiotic, it is preferable that the intake is about 0.5- 1.0 (BCFU) or more per day, more preferably about 10 BCFU's or more per day, more preferably about 50 BCFU's or more per day, more preferably about 100 BCFU's or more per day. The concentration of probiotic to be contained in an individual product serving is determined according to the amount of servings ingested per day. The concentration of probiotic present in the functional food composition can be less than about 1 BCFU's, about 1 to about 2 BCFU's, about 3 to about 5 BCFU's, about 5 to about 10 BCFU's, about 10 to about 20 BCFU's, about 20 to about 30 BCFU's, about 30 to about 40 BCFU's, about 40 to about 50 BCFU's, about 50 to about 60 BCFU's, about 60 to about 70 BCFU's, about 70 to about 80 BCFU's, about 80 to about 90 BCFU's, about 90 to about 100 or more BCFU's.

Under these criteria, the prebiotics can include, but are not limited to, galacto-oligosaccharides, inulin and oligofructose (fructo-oligosaccharide), isomalto-oligosaccharides, lactulose, lactosucrose, transgalacto-oligosaccharides, soybean oligosaccharides, tagatose, xylo-oligosaccharides, and combinations thereof.

The prebiotic can be an oligosaccharide fructan including, but not limited to, fructo-oligosaccharide and derivatives thereof of inulin (poly-D-fructose, itself derived from various plant products including bananas, onions, chicory root, garlic, asparagus, barley, wheat, jicama, leeks, etc.) or from transfructosylation action of a β-fructosidase as used, for example, by *Aspergillus* sp. metabolically active on sucrose. In other embodiments, the prebiotics are derived from plants and plant products including, but not limited to, artichoke, dandelion greens, bran, flour, legumes, oats, citrus fruits, apples, and root vegetables, cane sugar, etc. If desired, the prebiotic also serves as a dietary fiber source for the consumer or patient, but in certain conditions may be depleted away from being a nutritional source for the probiotic.

Compositions described herein include prebiotics and probiotics for maintenance and treatment of gastrointestinal health. If desired, additional compounds are included in the functional food composition to improve or maintain healthy conditions in the digestive tract. Additional compounds can be insoluble fibers and/or soluble fibers generally not specifically utilized by the probiotic; probiotic extracts (e.g., probiotic exudates and/or non-viable micro-organisms); digestive enzymes including, but not limited to, pancreatin, pancrelipase, papain, pepsin, diatase, ox bile, lactase, protease, amylase, lipase, bromelain, cellulose, malt diastase, glucoamylase, hemicellulose, beta-gluconase, phytase, trypsin, aminopeptidase, chymotrypsin, carboxypeptidase, elastase, maltase, sucrose, etc.; supplements including, but not limited to, glutamine, peppermint, artichoke, ginger, fennel, licorice root, anise seed; and compounds useful for immune health including, but not limited to, vitamins (A, E, C, D); minerals (zinc, selenium); plant derived materials such as Echinacea, ashwaganda, elderberry, etc.; and yeast extracts.

### Uses of prebiotic/probiotic functional foods

Edible transport compositions described herein are contemplated to provide a functional benefit to a consumer ("functional food"). Functional foods are formulated to provide an important nutritive food source that contributes to the prevention, management and/or treatment of chronic diseases. This benefit can be provided to a consumer when new ingredients or more of an existing ingredient are added to a food so that an added health benefit from the food product is gained. Probiotics are useful for a variety of health related conditions as well as general health maintenance. The effectiveness of probiotic treatment is enhanced through the use of a prebiotic that is consumed with a probiotic. The edible transport compositions as described herein increase the effective delivered concentration of such useful probiotics, and can be formulated for daily dietary supplements, preventative treatment or treatment of nosocomial infections, or for clinical applications treating systemic disease states.

Often the probiotics are recommended by physicians and nutritionists to treat, for example, candidiasis, infections by opportunistic bacteria establishing after trauma, stress, excessive alcohol, exposure to toxins, lactose intolerance, or the establishment of normal flora after treatment with antibiotics. In some embodiments, the edible transport compositions are used to assist the consumer's natural flora to re-establish and/or maintain normal concentrations in the stool and the digestive tract. Often the probiotics are recommended by physicians and nutritionists to treat, for example, candidiasis, infections by opportunistic bacteria establishing after trauma, or the establishment of normal flora after treatment with antibiotics. In some embodiments of the edible transport compositions, maintenance and/or treatment of gastrointestinal conditions or disorders is provided through a regimen of daily consumption at high concentrations (50 - 100 BCFU's) for one to two weeks followed by a maintenance treatment in a normal diet (1-10 BCFU's) to assist in the re-establishment normal health conditions for a consumer. Maintenance treatment in a normal daily diet (1-10 BCFU's) can be useful for increasing transit times of stool though the intestinal tract, decreasing intestinal gas production and bloating, increasing microflora levels in the stool, and/or improving digestion. Other conditions for which clinical changes and mitigation of intestinal symptoms are desired can take longer treatments at higher concentrations of the prebiotic and/or probiotic, for example 4 - 8 weeks.

Probiotics may play a role in the prevention of certain types of cancer (e.g. colon cancer) due to their ability to bind and metabolize carcinogenic substances such as heterocyclic amines. Lower rates of colon cancer have been correlated with high consumption of fermented dairy products. In some embodiments of the edible transport compositions, maintenance and/or treatment is provided through a daily regimen (1-10 BCFU's), which can be useful for contributing to the prevention of certain cancers.

Probiotics may play a role in benefitting the immune system by, for example, competing against pathogens though competitive inhibition (e.g. opportunistic bacteria such as *C*. *difficile*), enhancing phagocytosis by white blood cells, and enhancing or increasing the production of certain antibodies and/or T-lymphocytes. Modulation of the immune system by regulating inflammatory and hypersensitivity responses related to cytokine regulation and function is also found to be correlated to intake of probiotics, as is the management of diarrhea and inflammatory bowel disease. In some embodiments of the edible transport compositions, maintenance and/or treatment of immune response conditions or disorders is provided through a regimen of daily consumption at high concentrations (50 - 100 BCFU's) for one to two weeks followed by a maintenance treatment in a normal diet (1-10 BCFU's) to assist in the re-establishment of normal health conditions for a consumer. Maintenance treatment in a normal daily diet (1-10 BCFU's) can be useful for management of diarrhea and inflammatory bowel disease.

Probiotics may play a role in the treatment for systemic disorders such as high cholesterol and high blood pressure. Ingestion of sufficient levels of probiotics is correlated with lowering cholesterol through a mechanism thought to be involved with the breakdown of bile in the intestinal tract to a metabolite that is unable to be reabsorbed. Additionally, reduction in blood pressure has been correlated with intake of fermented dairy products. Thus, it is believed that angiotensin-converting-enzyme inhibitors or inhibitor like peptides are produced by the probiotic. Maintenance treatment in a normal daily diet (1-10 BCFU's) can be useful for management of systemic disorders such as high cholesterol and/or high blood pressure.

### XI. Overview of the β-glucan/probiotic functional food composition

The disclosure relates to functional food compositions that contain a β-glucan and a probiotic when, upon ingestion, promote immune responses and immunity related health and well-being. The functional food composition contains an edible or potable substance that is encapsulated in an edible membrane, and further contains a probiotic and a β-glucan. The functional food compositions described herein provides a convenient and improved way for individuals to consume probiotics and β-glucan in quantities needed to promote immunological health and well-being.

Generally, functional food compositions are intended to provide a healthy benefit to a consumer. The benefit can be qualitative, quantitative or subjective and include, for example, health promotion, disease prevention, etc. Some benefits include providing an important nutritive food source, vitamins and supplements, β-glucan or probiotics, which contribute to the prevention, management and treatment of chronic diseases or disorders. This health improvement benefit can also be provided to a consumer when additional ingredients or more of an existing ingredient are added to a food so that the benefit from the food product is gained. However, delivery systems for some beneficial health compounds including β-glucan and probiotics often have reduced bioavavailability, resulting in suboptimal quantities of these compounds being made available to the consumer.

The functional food compositions described herein provide for a significant improvement in the delivery, viability and concentration of β-glucan and probiotic compounds useful to a consumer. Within these compositions, the β-glucan and probiotic compounds can be compartmentalized within the edible or potable substance, the encapsulating membrane, or both. Upon mastication and ingestion, the β-glucan and probiotic are released from the functional food composition to provide an immediately available nutrition and edible source. The functional food compositions can thus be designed to have one or more β-glucan and probiotic compounds in an effective combination and concentration desirable for the specific needs of the consumer or patient, for example, the elderly, adult and children consumers, and medical patients.

The functional food compositions described herein are designed to provide β-glucan and probiotic compounds in biologically effective quantities superior to commonly used products. The compositions are particularly well-suited for providing a larger active ingredient load as well as being amenable for controlling the actual portion size for an intended health benefit. Functional food compositions as described herein can provide up to and above several times the suggested minimum dosages in a sample serving, or provide combinations of β-glucan and probiotic compounds, nutraceuticals and supplements up to and above several times the suggested minimum dosages in a single serving that is both convenient and appetizing. For example, an eight ounce serving of commercial yogurt in plastic containers commonly provides a microbial load of less than 1 billion colony forming units (BCFU), far below a bacterial load level sufficient to provide health benefits. The compositions described herein can be designed to provide up to 100 BCFU's. A functional food delivering a combination of β-glucan and probiotics in a single serving composition can contribute to the prevention, cessation or mitigation of a multitude of health related conditions and diseases. In addition, a general state of health and wellness can result from a healthy diet supplemented with β-glucan and live and/or heat killed probiotic compounds.

These functional food compositions also enhance the stability, gustatory experience, and delivery of functional biologically active compounds. Functional food compositions contain and protect ingestible/edible substances, such as ice cream, yogurts, etc., and probiotics or β-glucan by encapsulating the substance within edible or biodegradable membranes. The edible membranes of these compositions can be formed from various natural polymeric substances allowing different compositions to be easily transported and consumed.

Considerations for the choice of probiotic or probiotic combinations for use in the function food compositions are that, if desired, the probiotic remain viable until ingestion in a biologically effective concentration, and the probiotic be able to confer a health benefit to and/or use for treatment of a gastro-intestinal, immune or other systemic disorder in the host organism.

In some instances, heat-treated probiotics or probiotics that are otherwise rendered non-viable, and/or immune enhancing constituents derived from heat killed or non-viable probiotics, are preferred and are used in the compositions described herein. For example, heat killed *Lactobacillis* species have been shown to augment innate and acquired immunity in mammals.

In all instances, it is desirable that these food compositions (product) include effectual probiotics to enhance the immune response otherwise confer a health benefit to a host organism ingesting the probiotic.

β-glucans are major biological structural components of yeasts, fungi and certain bacteria. β-glucans are polysaccharides that can be obtained from natural sources, and consist of a backbone of (1,3)-β-glycosidic linked D-glucose subunits, and having regularly or irregularly placed β-(1,6)-linked (1,3)-β glycosidic linked D-glucose side chains of various lengths. The various side chain combinations can affect immunological effects due to resulting changes in the β-glucan solubility, mass, tertiary structure, branching patterns and electrostatic charge. Innate immune responses to invading pathogens depend primarily on the recognition of pathogen-associated molecular patterns (PAMPs) by pattern recognition receptors (PRR). β-glucans are known as a major PAMP for the PPR-mediated sensing of fungal infection. β-glucans polymers are able to bind to the dectin receptor (an important PRR for beta-glucans), to activate the dectin-1 signaling to enhance phagocytosis, presentation of macrophages and dendritic cells, and induces the adaptive immune response.

Generally, the probiotics and β-glucans as used herein are taken 1) to enhance the physical well-being or state of health of the end user, 2) as a health related supplement, 3) as supplements for enhancing immune response or mitigation of autoimmune responses, and/or 4) as supplements to maintain a healthy immune system. Diets supplemented with probiotics and β-glucans can also add to a higher quality or perceived quality of the health state of the end user.

Health benefit aspects contemplated for use of the β-glucans and probiotic functional food compositions described herein include relief from intestinal inflammation, beneficial pathogen and antigen response, decreased time of infection, decreased severity of infection, prevention of infection, etc. Health and wellness preventative treatment with probiotics includes decreased immunological response time, enhanced humoral immune response, stimulation of non-specific host resistance to microbial pathogens, and modulation of the host's immune responses to potentially harmful antigens, and regulation of hypersensitivity reactions, including statistically significant decreases in the onset of upper respiratory tract infections, demonstrated augmentation of acquired immunity, reduction in the severity of cold and flu symptoms, and reduction in nasal and ocular symptoms due to ragwood allergy. Additionally, β-glucans and probiotics may beneficially affect the host by augmenting the host intestinal microbial population beyond the amount already existing, thus possibly inhibiting unhealthy or harmful pathogens.

As described in more detail herein, the common probiotics include lactic acid bacteria, bifidobacteria, yeasts and bacilli. Often these probiotics are consumed as part of a fermented food source, (e.g., yogurt, soy yogurt, etc.) with added live microbiological cultures.

Measurable outcomes for general health maintenance can be determined quantitatively and/or qualitatively by monitoring, for example, a patient's or consumer's change in composition of intestinal microflora, decreased incidence of pathological infection, decreased intensity of pathological infection, decreased recovery time from pathological infection, and increased possibly feelings of well-being. For pretreatment/prevention of diseases, conditions and/or syndromes, the consumer or patient may show milder symptoms or no symptoms. In acute conditions for which the consumer or patient is using the functional food composition, for example diarrheal conditions (i.e. traveler's, antibiotic or viral) or infection from viruses related to the common cold, the outcome would be faster resolution and reduction in severity for treatment. In general, a reduction in discomfort related to infection or an immune response, and improvement in quality of life and ability to perform activities of daily living are all indicators of the health benefits when consuming the functional food compositions described herein.

### β-glucan and Probiotic compositions

Common probiotics include lactic acid bacteria, bifidobacteria, yeasts and bacilli, and are often consumed as part of a fermented food source, (e.g., yogurt, soy yogurt, etc.) with added live microbiological cultures. Probiotics in concentration and/or type contained within the functional food compositions included herein may be used in addition to natural levels of probiotics in the food source. Alternatively, probiotics as described herein may be used in food sources with no known natural probiotic levels. Inactivated probiotics, for example heat or chemically inactivated probiotics, having an antigenic effect may be used in the compositions described herein.

Probiotics for use in the foods described herein include, but are not limited to, *Aspergillus niger, Aspergillus oryzae, Bacillus coagulans, Bacillus lentus, Bacillus licheniformis, Bacillus pumilus, Bacillus subtilis, Bacteroides amylophilus, Bacteroides capillosus, Bacteroides ruminocola, Bacteroides suis, Bifidobacterium animalis, Bifidobacterium bifidum, Bifidobacterium breve, Bifidobacterium infantis, Bifidobacterium licheniformis, Bifidobacterium longum, Bifidobacterium pseudo longum, Bifidobacterium subtilus, Bifidobacterium thermophilum, Enterococcus cremoris, Enterococcus diacetylactis, Enterococcus faecium, Enterococcus thermophilus, Lactobacillus acidophilus, Lactobacillus amylovorus, Lactobacillus ansporogenes, Lactobacillus brevis, Lactobacillus buchneri, Lactobacillus bulgaris, Lactobacillus casei, Lactobacillus caucasicus, Lactobacillus cellobiosus, Lactobacillus crispatus, Lactobacillus curvatus, Lactobacillus delbrueckii, Lactobacillus farciminis, Lactobacillus fermentum, Lactobacillus gallinarum, Lactobacillus gasseri, Lactobacillus GG, Lactobacillus helveticus, Lactobacillus johnsonii, Lactobacillus kefir, Lactobacillus lactis, Lactobacillus plantarum, Lactobacillus reuteri, Lactobacillus rhamnosus* (casei), *Lactobacillus salivarius, Lactococcus lactis, Leuconostoc mesenteroides, Pediococcus acidilactici, Pediococcus acidilacticii, Pediococcus cerevisiae, Pediococcus pentosaceus, Propionbacterium freudenreichii, Propionibacterium shermanii, Saccharomyces boulardii, Saccharomyces cerevisiae, Shewanella colwelliana, Shewanella olleyana, Shewanella putrefaciens, Streptococcus cremoris, Streptococcus faecium, Streptococcus infantis, Streptococcus thermophilus, Vagococcus fluvialis, Bacillus coagulans* GBI-30, 6086, *Bifidobacterium animalis* DN 173 010, *Bifidobacterium animalis* subsp. *lactis* BB-12, *Bifidobacterium breve* Yakult, *Bifidobacterium infantis* 35624, *Bifidobacterium lactis* HN019 (DR10), *Bifidobacterium longum* BB536, *Bifidobacterium longum* subsp. *infantis* 35624, *Enterococcus* LAB SF 68, *Escerichia coli* Nissle 1917, *Lactobacillus acidophilus* NCFM, *Lactobacillus acidophilus* LA-5, *Lactobacillus acidophilus* NCFM, *Lactobacillus casei* subspecies *casei, Lactobacillus casei* DN-114 001, *Lactobacillus casei* CRL431, *Lactobacillus casei* F19, *Lactobacillus casei* Shirota, *Lactobacillus delbrueckii* subspecies *bulgaricus, Lactobacillus delbrueckii* subspecies *lactis, Lactobacillus paracasei* St11 (or NCC2461), *Lactobacillus johnsonii* La1 (Lj1), *Lactobacillus johnsonii* NCC533, *Lactobacillus plantarum* 299v, *Lactobacillus rhamnosus* ATCC 53013 (LG), *Lactobacillus rhamnosus* LB21, *Lactobacillus reuteri* ATCC 55730, *Lactobacillus salivarius* (UCC118), *Lactobacillus reuteri* SD2112, *Lactococcus lactis* L1A, *Lactococcus lactis* subspecies *lactis, Lactococcus lactis* subspecies *lactis* biovariant *diacetylactis, Lactococcus lactis* subspecies *cremoris, Leuconostoc mesenteroides* subspecies *cremoris, Leuconostoc mesenteroides* subspecies *dextranicum, Lactobacillus reuteri* Protectis (DSM 17938, daughter strain of ATCC 55730), *Saccharomyces cerevisiae boulardii, Saccharomyces cerevisiae boulardii* (lyo), *Lactobacillus bulgaricus, Streptococcus thermophiles, Bifidobacterium* spp, and any other strains, derivatives, and combinations thereof, including, but not limited to, *Lactobacillus rhamnosus* GR-1 & *Lactobacillus reuteri* RC-14, *Lactobacillus acidophilus* CL 1285 & *Lactobacillus casei* Lbc80r, *Lactobacillus rhamnosus (ATT SD5675)* & *Lactobacillus acidophilus* (STCC SD5221), VSL #3 (a combination of 1 strain of *Streptococcus* thermophiles, four strains of *Lactobacillus* species, & three *Bifidobacterium* species strains from Sigma-Tau Pharmaceuticals), *Lactobacillus acidophilus* NCFM & *Bifidobacterium bifidum* BB-12, *Lactobacillus acidophilus* CUL60 & *Bifidobacterium bifidum* CUL20, *Lactobacillus acidophilus* CL1285 & *Lactobacillus casei* LBC80R, *Lactobacillus helveticus* R0052 & *Lactobacillus rhamnosus* R0011, *Bacillus clausii* (strains O/C, NR, SIN and T), *Lactobacillus plantarum* HEAL 9 & *Lactobacillus paracasei* 8700:2, other genetic variants thereof, and combinations and other combinations thereof. In certain instances the probiotic may be heat killed or otherwise rendered non-viable and included in the functional food product described herein. In still other instances, constituents of the heat killed or otherwise rendered non-viable probiotic may be used in the functional foods described herein.

When the dosage or intake of the effective dose of the active ingredients of the functional food composition are indicated by the number (concentration) of the probiotic, it is preferable that the intake is about 0.5- 1.0 (BCFU) or more per day, more preferably about 10 BCFU's or more per day, more preferably about 50 BCFU's or more per day, more preferably about 100 BCFU's or more per day. The concentration of probiotic to be contained in an individual product serving is determined according to the amount of servings ingested per day. The concentration of probiotic present in the functional food composition can be less than about 1 BCFU's, about 1 to about 2 BCFU's, about 3 to about 5 BCFU's, about 5 to about 10 BCFU's, about 10 to about 20 BCFU's, about 20 to about 30 BCFU's, about 30 to about 40 BCFU's, about 40 to about 50 BCFU's, about 50 to about 60 BCFU's, about 60 to about 70 BCFU's, about 70 to about 80 BCFU's, about 80 to about 90 BCFU's, about 90 to about 100 or more BCFU's.

When the dosage or intake of the effective dose of the active ingredients of the functional food composition are indicated by the mass of the probiotic, it is preferable that the intake is about 1 mg to about 10 mg, about 10 mg to about 20 mg, about 20 mg to about 30 mg, about 30 mg to about 40 mg, about 40 mg to about 50 mg, about 50 mg to about 75 mg, about 75 mg to about 100 mg, about 100 mg to about 150 mg, about 150 mg to about 200 mg, about 200 mg to about 250 mg, about 250 mg to about 300 mg, about 300 mg to about 350 mg, about 350 mg to about 400 mg, about 400 mg to about 450 mg, about 450 mg to about 500 mg, about 500 mg to about 600 mg, about 600 mg to about 700 mg, about 700 mg to about 800 mg, about 800 mg to about 900 mg, about 900 mg to about 1000 mg, or more.

In the functional food compositions described herein, β-glucans to be used in the compositions can be derived or obtained from yeasts, fungi, certain bacterial species, plants such as seaweed, or combinations thereof. For example, yeasts such as baker's yeast *(Saccharomyces cerevisiae),* cereal grains including barley, rye, wheat, oats, and mushrooms (for example shiitake, maitake, reishi, shimeji and oyster varieties) are all common sources of β-glucan. Specifically, (1,3)-(1,6)-β-glucan has been shown to have clinical effect in boosting immune responses; however compositions with (1,3) β-glucan have also been shown to enhance immune responses. In certain embodiments, purified forms of (1,3)-(1,6)-β-glucan, for example from *Saccharomyces cerevisiae,* are used in the functional food composition.

Concentrations of the β-glucan used for an effective dose depend on the purity of the β-glucan, but can be daily doses of up to about 1 milligram (mg), 2 mgs, 5 mgs, 10 mgs, 20 mgs, 30 mgs, 40 mgs, 50 mgs, 75 mgs, 100 mgs, 200 mgs, 300 mgs, 400 mgs, 500 mgs, 600 mgs, 700 mgs, 800 mgs, 900 mgs, 1000 mgs, 1500 mgs, 2000 mgs, 2500 mgs, 3000 mgs, 3500 mgs, 4000 mgs, 4500 mgs, 5000 mgs. In certain embodiments, concentrations are desired to be about 200 mg to about 300 mg.

If desired, additional compounds are included in the functional food composition to improve or maintain healthy conditions in the digestive tract and/or enhance or modify the microflora and ecological biochemistry to optimize the effects of the probiotic and β-glucan composition. Additional compounds can be insoluble fibers and/or soluble fibers generally not specifically utilized by the probiotic; probiotic extracts (e.g., probiotic exudates and/or non-viable micro-organisms); digestive enzymes including, but not limited to, pancreatin, pancrelipase, papain, pepsin, diatase, ox bile, lactase, protease, amylase, lipase, bromelain, cellulose, malt diastase, glucoamylase, hemicellulose, beta-gluconase, phytase, trypsin, aminopeptidase, chymotrypsin, carboxypeptidase, elastase, maltase, sucrose, etc.; supplements including, but not limited to, glutamine, peppermint, artichoke, ginger, fennel, licorice root, anise seed; and compounds useful for immune health including, but not limited to, vitamins (A, E, C, D); minerals (zinc, selenium); plant derived materials such as Echinacea, ashwaganda, elderberry, etc.; and yeast extracts.

### Uses of β-glucan/probiotic functional foods

Edible transport compositions described herein are contemplated to provide a functional benefit to a consumer ("functional food"). Functional foods are formulated to provide an important nutritive food source that contributes to the prevention, management and/or treatment of chronic diseases, pathogenic infections and infectious conditions, and autoimmune regulation. This benefit can be provided to a consumer when new ingredients or more of an existing ingredient are added to a food so that an added health benefit from the food product is gained. Probiotics and β-glucans are useful for a variety of health related conditions as well as general health maintenance. Immune system effectiveness is enhanced with the combination of probiotic/β-glucan intake. The edible transport compositions as described herein increase the effective delivered concentration of such useful probiotics/β-glucan, and can be formulated for daily dietary supplements, preventative treatment of pathogenic conditions, treatment of nosocomial infections, for clinical applications treating systemic disease states, etc.

Probiotics and β-glucans are have been used as separate indications by physicians and nutritionists to treat, for example, candidiasis, infections by opportunistic bacteria and viruses establishing after trauma, stress, as an antioxidant, or the establishment of normal flora after treatment with antibiotics. In the functional foods described herein, it is preferred that combinations of live and/or non-viable probiotic are used in combination with a β-glucan in the final nutritional functional food product.

In some embodiments, the functional food compositions are used to assist in preventing or mitigating radiation burns. Often the probiotics are recommended by physicians and nutritionists to treat, for example, candidiasis, infections by opportunistic bacteria establishing after trauma, or the establishment of normal flora after treatment with antibiotics. In certain embodiments the functional food described herein can be used to support a cancer treatment regimen (being effective in promoting macrophage generation targeting cancer cells) or for the promotion of the generation of immunocytes in bone marrow.

Probiotics and β-glucans may also play a role in benefitting the immune system by, for example, competing against pathogens though competitive inhibition (e.g. opportunistic bacteria such as *C*. *difficile*) via enhancing natural flora, enhancing phagocytosis by white blood cells, and enhancing or increasing the production of certain antibodies and/or T-lymphocytes. Modulation of the immune system by regulating inflammatory and hypersensitivity responses related to cytokine regulation and function is also found to be correlated to intake of probiotics and β-glucans, as is the management of diarrhea and inflammatory bowel disease.

The functional food described herein may play a role in the treatment for systemic disorders such as high cholesterol and high blood pressure. Ingestion of sufficient levels of probiotics is correlated with lowering cholesterol through a mechanism thought to be involved with the breakdown of bile in the intestinal tract to a metabolite that is unable to be reabsorbed. Additionally, reduction in blood pressure has been correlated with intake of fermented dairy products. Thus, it is believed that angiotensin-converting-enzyme inhibitors or inhibitor like peptides are produced by the probiotic. Maintenance treatment in a normal daily diet (1-10 BCFU's) can be useful for management of systemic disorders such as high cholesterol and/or high blood pressure.

### Membranes encapsulating compositions

Embodiments of transport system described herein can have, e.g., varying shell or membrane thickness, one or more of a variety of chemical constituents, varying numbers of membranes, various consumable payloads, various shapes, and are constructed from various shell/membrane properties to provide a variety of flavors and textures and membrane characteristics. See, for example, PCT International Publication No. WO 2011/103594 and PCT International Application No. PCT/US2013/023500.

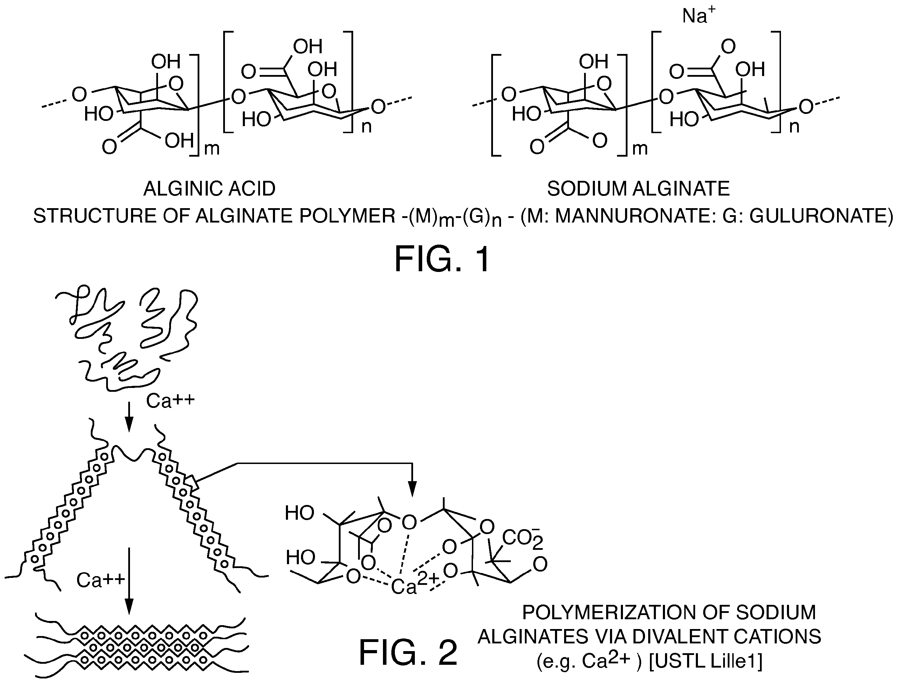

Membranes and shells of the functional food compositions may be made by using any one of many edible and/or biodegradable polymers. Figure 1 illustrates alginate (alginic acid) as an example of a polymer that can be used in forming a membrane of transport systems. Alginate is an anionic, polymeric polysaccharide, widely present in the cell walls of brown algae. It is a copolymer -(M)ₘ-(G)ₙ-segments composed of mannuronate M (mannuronic acid) and guluronate G (guluronic acid) monomeric subunits. The values of m and n, the ratio m/n, and the space distribution between M and G (i.e. presence of consecutive G-subunits and M-subunits, or randomly organized subunits) all play key roles in the chemical and physical properties of the final membrane. To induce rapid gelation by electrostatic cross-linking, the naturally present Na⁺ ions are removed and replaced by divalent cations (e.g., Ca²⁺ or another multi-valent cation such as Mg²⁺; Figure 2).

Generally, membrane encapsulated compositions are constructed that use various particles, particulates and polymers, in combination or separately, to create desired properties of strength, stability, permeability, edibility and biodegradability for the transport systems to be easily moved and consumed.

For example, functional food compositions can be encased in a polysaccharide membrane, for example, an alginate membrane. Ingestible particles can be embedded in the membrane to improve the physical, chemical and/or physico-chemical performance characteristics suitable for particular payloads (i.e. edible or potable substances), including, but not limited to, diffusion characteristics, pore size of the membrane, elasticity, etc. When particles are charged and possess the same charge state as other membrane polymers or particulates, membrane component concentrations can vary (for example, decreasing the membrane polymer concentration and increasing the membrane particulate concentration) while maintaining or optimizing membrane performance. When particles carry the opposite charge state as alginate polymers or particulates, the need for a calcium solution or another multivalent ion can be minimized or eliminated by using particles to bind with alginates or another charged polymer. For non-alginate based systems, combinations of homogenous particles can be used to encapsulate the edible material, or can be used in combination with polymers at lower weight %-by-mass than the particles (for example, less than 80%, less than 70%, less than 60%, less than 50%, less than 40%, less than 30%, less than 20%, less than 10% polymer). In some applications, a thinner membrane is desirable to encapsulate a larger quantity of ingestible material, which may have further advantages of taste and texture. Particles contemplated herein include large food particles, for example greater than 1 millimeter (linseeds, sesame seeds, poppy seeds, chia seeds, chopped or pulverized foods including fruits, fruit skins, vegetables, etc.), small grains, and pulverized seeds, nuts, etc. In some applications, compositions use particulates less than about 1 millimeter.

Generally, polysaccharide polymers are used as the membrane polymer. Polysaccharide polymer based membranes are porous, with porosity determined by the chemical content and 2- and 3-dimensional geometry of the polymeric structure of the membrane, for example the structure of the polysaccharide chain. As described herein, various physical and chemical characteristics of the particulates are matched to the membrane structure and chemistry to achieve a desired effect, such as increased impermeability, elasticity, membrane strength-to-weight ratio, color, syneresis, etc. The membrane can have a strength sufficient to support a volume of water, for example 1 cubic centimeter (cc), 5 cc, 10 cc, 20 cc, 30 cc, 40 cc, 50 cc, 100 cc, 200 cc, 300 cc, 400 cc, 500 cc, 600 cc, 700 cc, 800 cc, 900 cc, 1000 cc, 1200 cc, 1400 cc, 1600 cc, 1800 cc, 2000 cc, 3000 cc, 4000 cc, 5000 cc, 6000 cc, 7000 cc, 8000 cc, 9000 cc, 10000 cc, or more, without rupturing, and when handled or transported. The membrane is preferably a continuous membrane, fully encapsulating an edible or potable substance such that the edible or potable substance is fully contained and does not leak out from within encapsulation due to mechanical stress, rupture, shearing, etc. The cross-linked matrix can have a thickness from about 10 microns to about 200 millimeters. The edible or potable substance can be coated and/or embedded in a plurality of membranes.

The particulates used for the membrane can be about 0.01microns, at about 0.1 microns, at about 0.1 to 1.0 microns, at about 0.1 to 10 microns, at about 0.1 to 100 microns, at about 0.01 to about 1 millimeter or to about 3 millimeters, or at about 0.1 to about 1 millimeter or to about 3 millimeters. The size of the particulates may be important for embedment characteristics into the porous structure of the membrane.

In some applications, it may be desirable that some of the particulates are incorporated into the membrane and a layer of particulates remain unincorporated, forming a layer next to a membrane or between two or more membrane layers. The additional particulate layer can be engineered to alter, for example, permeability, elasticity, strength, durability, syneresis, hygroscopy, hydrophobicity, etc., changes across and within membrane layers. Thus, the chemical nature of the particulates, for example if a hydrophobic particulate is used, can enhance the impedance of liquid diffusion across an inner layer to an outer layer surface boundary. In other aspects, particulates can be layered so that the particulate layer has multiple effects, for example an inner impermeability layer, a middle flavor/texture/payload (e.g. a pharmaceutical or supplement) layer, and an outer strength improving layer.

Particles as described herein can be both in the edible or potable substance and in the membrane. The particles in the membrane and in the edible or potable substance can be the same particles or different particles, can be uniform size or exhibit a range of sizes, and/or exhibit similar or different physical-chemical characteristics.

Various membrane polymers are contemplated for use in the membrane forming layers. Polysaccharide polymers contemplated herein include, but are not limited to, shellac, various fibers and hydrocolloids such as alginate, an agar, a starch, a gelatin, carrageenan, xanthan gum, gellan gum, galactomannan, gum arabic, a pectin, a milk protein, a cellulosic, gum tragacanth and karaya, xyloglucan, curdlan, a cereal β-glucan, soluble soybean polysaccharide, a bacterial cellulose, a microcrystalline cellulose, chitosan, inulin, an emulsifying polymer, konjac mannan/konjac glucomannan, a seed gum, and pullulan. Combinations of these polysaccharides are also contemplated herein.

Other membrane compounds considered for use as structure forming compounds to modify or be used in combination with a polymer-based membrane (for example, a membrane consisting of a polysaccharide) include bagasse, tapioca, chitosan, polylactic acid, processed seaweed, chocolate, starch, gum arabic, cellulose based fibers, natural and synthetic amino acids and polymers thereof, proteins and sugars/sugar derivatives, etc. Combinations of these compounds and compositions are also contemplated herein.

One or more of a variety of ions can be used to polymerize the membrane and for related chemical processes. In, for example, the alginate polysaccharide membrane, ions are used to form cross-linkages between and among individual polymer strands. Various ion/counter ion salt complexes are contemplated for use herein, including, but not limited to, divalent cations such as calcium, potassium, magnesium, manganese, iron, zinc; trivalent cations including, but not limited to, manganese and iron; and salts thereof including, but not limited to, calcium lactate and calcium chloride.

In certain applications micelles can be formed within membranes and between membrane layers and/or between the inner membrane and the edible or potable substance. Micelles can alter the taste experience or mouth feel for the final encased product. Additionally, micelles engineered into the final membrane coated product may contain other ingestibles including sweeteners, flavors (fruits, herbs and spices, etc.), herbal extracts, energy supplements, dietary supplements, pharmaceuticals, over the counter drugs, sleep aids, appetite suppressants, weight gain agents, antioxidants, nutraceuticals, confections, etc., and combinations thereof.

Useful characteristics of membranes described herein can provide sufficient barriers to environments and environmental changes, thereby protecting food compositions, probiotics and prebiotics, by, for example, prevention or attenuation of dehydration, providing oxygen barriers, thermal insulation, etc., that increase the length of time the probiotic and functional food as a whole remains viable.

### Encapsulated compositions

Functional foods are desired to provide health and medical benefits, including the prevention and treatment of disease. Other factors also are considered to create a product that is commercially available (e.g. longer shelf life, product stability, etc.) and elicits a pleasurable experience for the consumer (e.g., flavor, ease of consumption, mouthfeel, etc.). Therefore, some considerations for the choice of food composition used as a carrier for the bioavailable compound include consumer taste preference, sufficiency to support desired concentrations of bioavailable compounds, compatibility of chemical environment between composition and bioavailable compound, compatibility of flavors of bioavailable compound and composition, and health and/or nutritional content of the composition.

Viability considerations for the choice of probiotic microorganism are influenced by the environment provided by the food composition in which the probiotic is delivered and ingested. Such considerations include, for example, a physiologically active state within the edible product, environmental temperature, pH, water activity, oxygen level, toxicity of the ingredients of the food product, potential for shear forces encountered during standard food production, and deleterious effects from freeze/thaws during storage and transport. Choice of probiotic can therefore influence choice of food composition, and vice versa, for the composition used as a delivery vehicle.

Taste to the consumer is important and can provide an enjoyable gustatory experience. Enjoyable food compositions and flavors can increase compliance of usage by consumers (e.g. children and elderly) and patients under clinical administration of the functional food. For example, typical flavorings for use in the compositions described herein include chocolate, vanilla, mints, natural fruit flavors, etc.

Edible and potable substances contemplated for the functional food include, but are not limited to, frozen dairy products such as frozen yogurts, ice creams, sorbets, gelatos, etc., other dairy products such as yogurt, fruits, vegetables, meat, a carbohydrate food product, botanicals, confections, and combinations thereof. The substances can be in natural form, puree form, frozen, soft, etc.

Other edible and potable substances that can be used as carriers of the bioavailable compounds include liquids such as water, alcoholic beverages and mixed drinks, juices, coffee and tea products, soft drinks, liquid confectionary beverages, and combinations thereof.

Additionally, various vitamins, minerals, supplements and nutraceuticals can fortify the composition, in addition to the bioavailable compounds used to make the functional food. In general, nutraceuticals provide a physiological benefit or provide protection against chronic disease. Such products may range from isolated nutrients, dietary supplements and specific diets to genetically engineered foods, herbal products, and processed foods such as cereals, soups, and beverages.

### Preparation of an edible, membrane encased composition

A membrane can be engineered for various functional food applications by being, for example, stronger, thinner/thicker, or taste a particular way, with methods in addition to adjusting the properties of an alginate solution. Adding suspended particles of food, nutraceuticals, compounds beneficial to gastrointestinal health, or other particles at least partially insoluble in water can achieve desirable properties important to the consumer and patient.

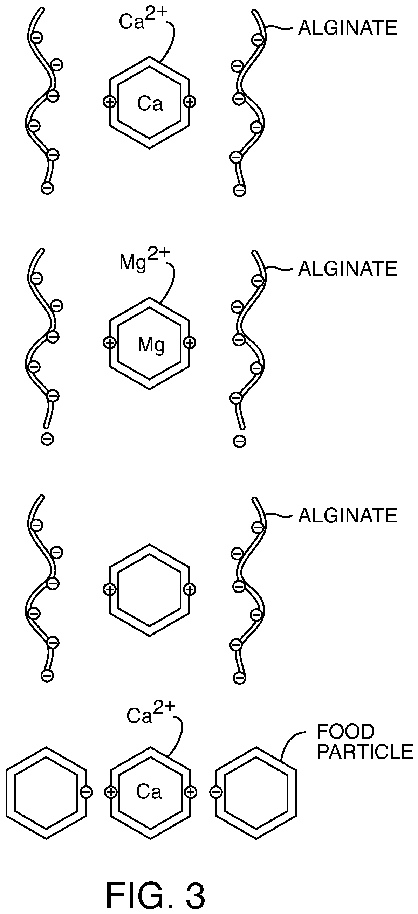

Often the added particles will be charged (i.e., most particle surfaces have some charge or zeta potential). This charge can be modified by the way each particle is created, its size, and the nature of the particle surface. Surfactants can be added to enhance the charged nature and the ionic atmosphere of the water can also be modified beneficially. When in solution (e.g., alginate or an aqueous medium), these particles can undergo strong or weak associations with alginate. When in contact with calcium, for example, particles will form with alginate a gelled membrane through interaction of the calcium and food particles trapped within the membrane, possibly strengthening it, improving flavor, etc. Figure 3 schematically illustrates the interaction between positively charged particles (e.g., Ca²⁺ or Mg²⁺) with negatively charged alginate or food particles.

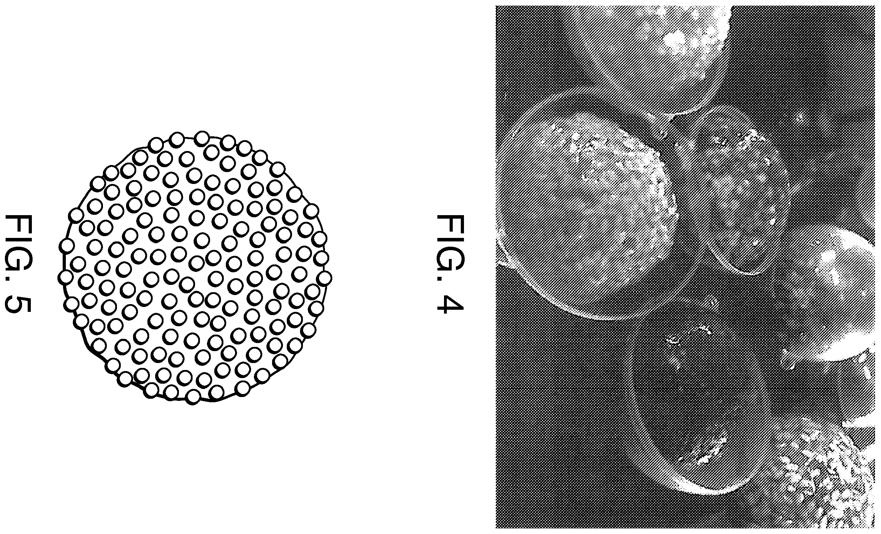

Figure 4 illustrates various food compositions having membrane layers containing different particles (e.g., edible particles). By way of example, membrane layers can include differently sized particles, different types of particles, or different orientations or configurations of particles. The functional food compositions can be sized to various diameters, from greater than 1.5 centimeters to 2 centimeters, 3 centimeters, 4 centimeters, 5 centimeters, 7.5 centimeters, 10 centimeters, 15 centimeters, or 20 centimeters, or greater. Additionally, the functional food compositions can be enclosed in various shells for packaging, transportation, or storage.

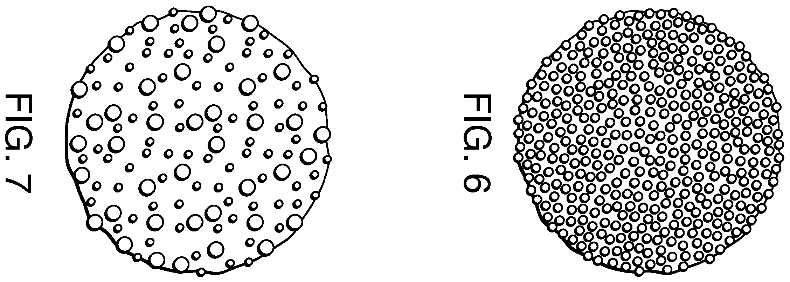

Referring to Figures 5, 6 and 7 a membrane layer around an ingestible substance can include larger and/or smaller particles suspended in the alginate polymer matrix, providing structural stability to the membrane and helping reduce deformation of the membrane. Such a membrane can have an unusual (e.g., non-spherical) shape. Additionally, particles can reduce evaporation of the aqueous portion of the membrane and/or the fluid inside the membrane, and provide a more rigid and/or less sticky surface for holding the composition.

The membrane layer having both large and small particles has been shown to produce better particle packing and arrangement within the membrane layer, possibly better structural integrity, reduced water evaporation from the membrane or the fluid contained therein, and forming more useful textures than membrane layers having only large or small particles.

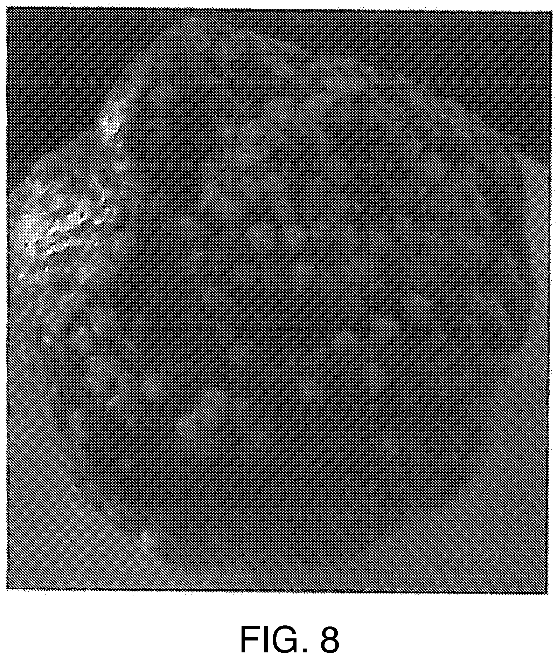

Functional food compositions can be formed as non-spherical, non-uniform shapes. Referring to Figure 8, a membrane can include ridges or features for aesthetic and/or structural purposes. The functional food composition can be made to resemble naturally occurring objects (e.g., fruits and vegetables) to enhance the appearance of the product as a natural alternative to various pills shaped supplements and the like

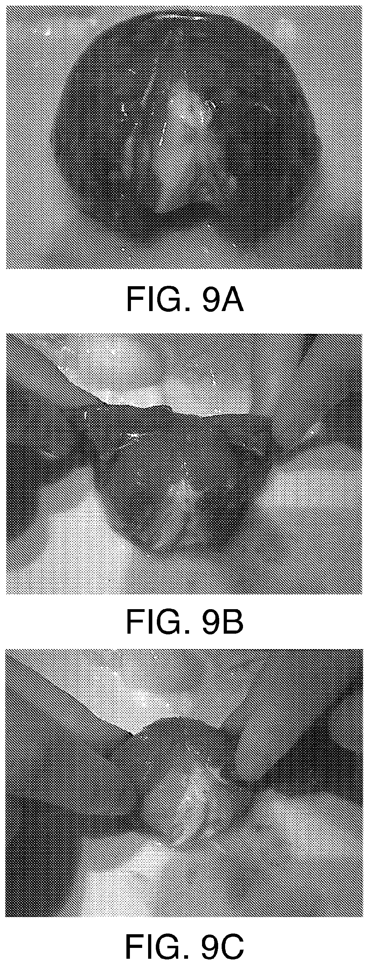

Referring to Figure 9a-c, some functional food compositions can be made with multiple layers of membranes with particles embedded therein, and further have particulate layers between the membrane matrix layers, encasing an edible or potable substance. For example, a probiotic can be embedded in one membrane, and the prebiotic can be embedded in another membrane. In other alternatives, more than one probiotic or prebiotic can be used and isolated or embedded in separate membranes of the multi-membrane product.

### Example 1-Gastrointestinal health functional food composition

Membrane layer and inner compositions to be used in the functional nutrition transport systems can include various ingredients to achieve different products (e.g., different flavors, textures and ingredients addressing specific health purposes) based on the requirements or needs of the intended end user as well as recommended daily values or values sufficient to achieve a health benefit. Three activities prebiotic and probiotics are thought to participate in are changing the microbiota, fermentation producing organic acids, producing a health benefit to the host. Various health benefits include improved digestion, reduced intestinal gas, increased stool transit time, reduced diarrhea, improved immune function, increased probiotic CFU counts in stool, etc. An example of membrane layer composition and inner composition to be enclosed by the membrane are provided below in Tables 1a/b for a prebiotic/probiotic nutritional product for use in enhancing digestive health and/or immunity.

**Table 1a**

| Inner Frozen Yogurt | |
|---|---|
| Ingredient | Amount per individual edible transport vessel |
| Frozen yogurt (*e.g*. vanilla) | 5 - 15 g |
| Howaru *Dophilus* (Danisco) | 100 BCFU |
| Howaru *Bifidus* (Danisco) | 100 BCFU |
| BC30 (*B. coagulans,* Ganaden Biotech) | 15 BCFU |

**Table 1b**

| Membrane | |
|---|---|
| Ingredient | Amount per individual edible transport vessel |
| Sodium Alginate | 0.01 - 0.4 g |
| Fructo-Oligosaccharide, Galacto-oligosaccharide, Inulin, chicory root, etc. (*eg*. VitaFiber) | 3 g (up to about 30 grams, optionally) |
| Water | 1.0 - 4.0 |
| Calcium lactate | < 0.5 g |
| Fruit/Flavoring | 1.0 - 15 g |
| Sweetener | 0.25 - 1.0 g |
| Sodium Citrate (optional) | 0.025 - 0.1 g |
| Stabilizer | 0.025 - 0.1 g |

### Example 2-Integument health functional food composition (Not according to the invention)

Membrane layer and inner compositions to be used in the functional food compositions can include various ingredients to achieve different products (e.g., different flavors, textures and ingredients addressing specific health purposes) based on the requirements or needs of the intended end user as well as recommended daily values or values sufficient to achieve a health benefit, as described herein. An example of membrane layer composition and inner composition to be enclosed by the membrane are provided below in Tables 2a/b for a bioflavonoid, carotene and elastic tissue component product, to deliver daily recommended or desired concentrations bioavailable compounds for beauty and health and wellness. Concentrations can be varied to provide the same concentrations in more than one serving.

**Table 2a**

| Inner Ice Cream | |
|---|---|
| Ingredient | Amount per serving |
| Non- or low-fat ice cream (vanilla, chocolate) | 5 - 20 g |
| Calcium lactate pentahydrate | 155 mgs |
| Magnesium amino acid chelate | 155 mgs |
| Zinc gluconate dihydrate | 7 mgs |

**Table 2b**

| Membrane | |
|---|---|
| Ingredient | Amount per serving |
| Sodium Alginate | 0.01 - 0.4 g |
| Water | 3.0 - 12.0 g |
| Calcium lactate | < 0.5 g |
| Chocolate (cacao paste, optionally bar chocolate) | 1.0 - 4.0 g |
| Sweetener (granulated sugar, trimoline, dehydrated glucose syrup) | 0.6 - 2.4 g |
| Cream | 0.3 - 1.2 g |
| Sodium Citrate (optional) | 0.025 - 0.1 g |
| Cocoa flavanol | 3-300 mgs cocoa flavanol containing about 75 % epicatechin and 25% catechin |
| Collagen | 1250 mgs |
| Lutein | 25 mgs |
| Silicic acid (Horsetail) | 70 mgs |
| Biotin | 1.3 mgs |
| Vitamin C | 10 mgs |

### Example 3-Weight loss functional food composition (Not according to the invention)

Membrane layer and inner compositions to be used in the functional food compositions can include various ingredients to achieve different products (e.g., different flavors, textures and ingredients addressing specific health purposes) based on the requirements or needs of the intended end user as well as recommended daily values or values sufficient to achieve a health benefit, as described herein. An example of membrane layer composition and inner composition to be enclosed by the membrane are provided below in Tables 3a/b for a weight loss product, to deliver daily recommended or desired concentrations for weight loss and treatment of weight related related disorders and diseases. Concentrations can be varied to provide the same concentrations in more than one serving.

**Table 3a**

| Inner Ice Cream | |
|---|---|
| Ingredient | Amount per serving |
| Low fat, low cholesterol ice | 5 - 20 g |
| cream (*e.g*. mint) | |
| Appethyl | 0.1-5.0 g |

**Table 3b**

| Membrane | |
|---|---|
| Ingredient | Amount per serving |
| Sodium Alginate | 0.01 - 0.4 g |
| Water | 3.0 - 12.0 g |
| Calcium lactate | < 0.5 g |
| Chocolate (cacao paste, optionally bar chocolate) | 1.0 - 4.0 g |
| Sweetener (granulated sugar, trimoline, dehydrated glucose syrup) | 0.6 - 2.4 g |
| Cream | 0.3 - 1.2 g |
| Sodium Citrate (optional) | 0.025 - 0.1 g |
| Stabilizer | 0.025 - 0.1 g |
| Appethyl | 0.1 - 5.0 g |

### Example 4-Skeletal health functional food composition (Not according to the invention)

Membrane layer and inner compositions to be used in the functional food compositions can include various ingredients to achieve different products (e.g., different flavors, textures and ingredients addressing specific health purposes) based on the requirements or needs of the intended end user as well as recommended daily values or values sufficient to achieve a health benefit, as described herein. An example of membrane layer composition and inner composition to be enclosed by the membrane are provided below in Tables 4a/b for a skeletal health product, to deliver daily recommended or desired concentrations for bone maintenance. Concentrations can be varied to provide the same concentrations in more than one serving.

**Table 4a**

| Inner Ice Cream | |
|---|---|
| Ingredient | Amount per individual serving |
| Ice cream (e.g. chocolate) | 5 - 20 g |

**Table 4b**

| Membrane | |
|---|---|
| Ingredient | Amount per individual serving |
| Sodium Alginate | 0.01 - 0.4 g |
| Calcium citrate malate | 200 - 1200 mg |
| Magnesium citrate | 80 - 320 mg |
| Vitamin D | 500-4000 IU |
| Water | 3.0 - 12.0 |
| Calcium lactate | < 0.5 g |
| Chocolate (cacao paste, optionally bar chocolate) | 1.0 - 4.0 g |
| Sweetener (granulated sugar, trimoline, dehydrated glucose syrup) | 0.6 - 2.4 g |
| Cream | 0.3 - 1.2 g |
| Sodium Citrate (optional) | 0.025 - 0.1 g |
| Stabilizer | 0.025 - 0.1 g |

### Example 5-Cardiovascular health functional food composition (Not according to the invention)

Membrane layer and inner compositions to be used in the functional food compositions can include various ingredients to achieve different products (e.g., different flavors, textures and ingredients addressing specific health purposes) based on the requirements or needs of the intended end user as well as recommended daily values or values sufficient to achieve a health benefit, as described herein. An example of membrane layer composition and inner composition to be enclosed by the membrane are provided below in Tables 5a/b for a cardiovascular health composition to deliver daily recommended or desired concentrations for bioflavonoids, carotenes and essential fatty acid compounds. Concentrations can be varied to provide the same concentrations in more than one serving.

**Table 5a**

| Inner Ice Cream | |
|---|---|
| Ingredient | Amount per serving |
| Non- or low-fat ice cream (vanilla, chocolate) | 5 - 20 g |
| Life's Microencapsulated DHA | 1000 mgs |

**Table 5b**

| Membrane | |
|---|---|
| Ingredient | Amount per serving |
| Sodium Alginate | 0.01 - 0.4 g |
| Water | 3.0 - 12.0 g |
| Calcium lactate | < 0.5 g |
| Chocolate (cacao paste, optionally bar chocolate) | 1.0 - 4.0 g |
| Sweetener (granulated sugar, trimoline, dehydrated glucose syrup) | 0.6 - 2.4 g |
| Cream | 0.3 - 1.2 g |
| Sodium Citrate (optional) | 0.025 - 0.1 g |
| Stabilizer | 0.025 - 0.1 g |
| Lycopene | 3 milligrams |
| Cocoa flavanols | 250 milligrams |

### Example 6-Joint and cartilage health functional food composition (Not according to the invention)

Membrane layer and inner compositions to be used in the functional food compositions can include various ingredients to achieve different products (e.g., different flavors, textures and ingredients addressing specific health purposes) based on the requirements or needs of the intended end user as well as recommended daily values or values sufficient to achieve a health benefit, as described herein. An example of membrane layer composition and inner composition to be enclosed by the membrane are provided below in Tables 6a/b for a joint and cartilage health product, to deliver daily recommended or desired concentrations for joint and cartilage maintenance and treatment of related disorders and diseases. Concentrations can be varied to provide the same concentrations in more than one serving.

**Table 6a**

| Inner Ice Cream | |
|---|---|
| Ingredient | Amount per individual serving |
| Ice cream (e.g. chocolate) | 5 - 20 g |

**Table 6b**

| Membrane | |
|---|---|
| Ingredient | Amount per serving |
| Sodium Alginate | 0.01 - 0.4 g |
| Water | 3.0 - 12.0 |
| Calcium lactate | < 0.5 g |
| Chocolate (cacao paste, optionally bar chocolate) | 1.0 - 4.0 g |
| Sweetener (granulated sugar, trimoline, dehydrated glucose syrup) | 0.6 - 2.4 g |
| Cream | 0.3 - 1.2 g |
| Sodium Citrate (optional) | 0.025 - 0.1 g |
| Stabilizer | 0.025 - 0.1 g |
| Glucosamine | Up to 2000 mg |
| Chondroitin | Up to 2000 mg |

### Example 7-Multivitamin functional food composition (Not according to the invention)

Membrane layer and inner compositions to be used in the functional food compositions can include various ingredients to achieve different products (e.g., different flavors, textures and ingredients addressing specific health purposes) based on the requirements or needs of the intended end user as well as recommended daily values or values sufficient to achieve a health benefit, as described herein. An example of membrane layer composition and inner composition to be enclosed by the membrane are provided below in Tables 7a/b for a multivitamin product, to deliver daily recommended or desired concentrations for multivitamins and treatment for malnutrition and dietary imbalances. Concentrations can be varied to provide the same concentrations in more than one serving.

**Table 7a**

| Inner Ice Cream | |
|---|---|
| Ingredient | Amount per serving |
| Chocolate ice cream | 5 - 20 g |

**Table 7b**

| Membrane | |
|---|---|
| Ingredient | Amount per serving |
| Sodium Alginate | 0.01 - 0.4 g |
| Water | 3.0 - 12.0 g |
| Calcium lactate | < 0.5 g |
| Chocolate (cacao paste, optionally bar chocolate) | 1.0 - 4.0 g |
| Sweetener (granulated sugar, trimoline, dehydrated glucose syrup) | 0.6 - 2.4 g |
| Cream | 0.3 - 1.2 g |
| Sodium Citrate (optional) | 0.025 - 0.1 g |
| Stabilizer | 0.025 - 0.1 g |
| AlternaVites ™ (for kids) | 2.5 g |

AlternaVites for kids is a commercially available multivitamin supplement composition labeled to contain 50% RDA vitamin A, 100% RDA vitamin C, 125% RDA vitamin D, 100% RDA vitamin E, 100% RDA thiamin, 100% RDA riboflavin, 100% RDA niacin, 1005 RDA vitamin B6, 100% RDA folic acid, 100% RDA vitamin B12, 17% RDA biotin, 100% RDA pantothenic acid, 15% RDA calcium, 100% RDA iodine, 10% RDA magnesium, 100% RDA zinc, and 50% RDA manganese, with other sweeteners and flavorings.

### Example 8-Essential fatty acids functional food composition (Not according to the invention)

Membrane layer and inner compositions to be used in the functional food compositions can include various ingredients to achieve different products (e.g., different flavors, textures and ingredients addressing specific health purposes) based on the requirements or needs of the intended end user as well as recommended daily values or values sufficient to achieve a health benefit, as described herein. An example of membrane layer composition and inner composition to be enclosed by the membrane are provided below in Tables 8a/b for an essential fatty acid and dietary fiber product, to deliver daily recommended or desired concentrations for DHA for developmental health and dietary fiber for gastrointestinal health. Concentrations can be varied to provide the same concentrations in more than one serving.

**Table 8a**

| Inner Ice Cream | |
|---|---|
| Ingredient | Amount per serving |
| Non-fat (vanilla) ice cream | 5 - 20 g |
| Life's Microencapsulated DHA | 500 mgs |

**Table 8b**

| Membrane | |
|---|---|
| Ingredient | Amount per serving |
| Sodium Alginate | 0.01 - 0.4 g |
| Water | 3.0 - 12.0 g |
| Calcium lactate | < 0.5 g |
| Chocolate (cacao paste, optionally bar chocolate) | 1.0 - 4.0 g |
| Sweetener (granulated sugar, trimoline, dehydrated glucose syrup) | 0.6 - 2.4 g |
| Cream | 0.3 - 1.2 g |
| Sodium Citrate (optional) | 0.025 - 0.1 g |
| Stabilizer | 0.025 - 0.1 g |
| Dietary fiber (e.g. inulin) | 3 grams |

### Example 9-Branched-chain amino acid functional food composition, (Not according to the invention)

Membrane layer and inner compositions to be used in the functional food compositions can include various ingredients to achieve different products (e.g., different flavors, textures and ingredients addressing specific health purposes) based on the requirements or needs of the intended end user as well as recommended daily values or values sufficient to achieve a health benefit, as described herein. An example of membrane layer composition and inner composition to be enclosed by the membrane are provided below in Tables 9a/b for BCAA (and glutamine) functional food product, to deliver daily recommended or desired concentrations bioavailable compounds for general health and wellness. Concentrations can be varied to provide the same concentrations in more than one serving.

**Table 9a**

| Inner ice cream | |
|---|---|
| Ingredient | Amount per serving |
| Ice cream | 5 - 20 grams |
| BCAA composition (Vitamin Shoppe Body Tech BCAA + Glutamine product) | 3.5 grams |

**Table 9b**

| Membrane | |
|---|---|
| Ingredient | Amount per serving |
| Sodium Alginate | 0.01 - 0.4 g |
| Water | 3.0 - 12.0 g |
| Calcium lactate | < 0.5 g |
| Sweetener (granulated sugar, trimoline, dehydrated glucose syrup) | 0.6 - 2.4 g |
| Sodium Citrate (optional) | 0.025 - 0.1 g |
| Stabilizer | 0.025 - 0.1 g |
| Mineral supplement blend, powder form | 10%-20% RDA |

### Example 10-Antioxidant functional food composition (Not according to the invention)

Membrane layer and inner compositions to be used in the functional food compositions can include various ingredients to achieve different products (e.g., different flavors, textures and ingredients addressing specific health purposes) based on the requirements or needs of the intended end user as well as recommended daily values or values sufficient to achieve a health benefit, as described herein. An example of membrane layer composition and inner composition to be enclosed by the membrane are provided below in Tables 10a/b for an antioxidant functional food product, to deliver daily recommended or desired concentrations bioavailable compounds for general health and wellness. Concentrations can be varied to provide the same concentrations in more than one serving.

**Table 10a**

| Inner frozen yogurt | |
|---|---|
| Ingredient | Amount per serving |
| Frozen yogurt | 5 - 20 grams |
| Fiber | 3-5 grams |

**Table 10b**

| Membrane | |
|---|---|
| Ingredient | Amount per serving |
| Sodium Alginate | 0.01 - 0.4 g |
| Water | 3.0 - 12.0 g |
| Calcium lactate | < 0.5 g |
| Sweetener (granulated sugar, trimoline, dehydrated glucose syrup) | 0.6 - 2.4 g |
| Sodium Citrate (optional) | 0.025 - 0.1 g |
| Stabilizer | 0.025 - 0.1 g |
| Vita-Veggie® High-ORAC fruit and veggie blend | 1-2 grams |
| Fresh fruit particles, chopped or minced | 2 grams |

### Example 11-Functional food composition

Membrane layer and inner compositions to be used in the functional nutrition transport systems can include various ingredients to achieve different products (e.g., different flavors, textures and ingredients addressing specific health purposes) based on the requirements or needs of the intended end user as well as recommended daily values or values sufficient to achieve a health benefit. Various health benefits include improved immune response, improved vaccination response, modulated hypersensitive immune response, cancer treatment, fungal infection treatment, etc. An example of membrane layer composition and inner composition to be enclosed by the membrane are provided below in Tables 11a/b for a β-glucan/probiotic nutritional product for use in enhancing immunity.

**Table 11a**

| Inner Frozen Yogurt | |
|---|---|
| Ingredient | Amount per individual serving |
| Frozen yogurt (*e.g*. vanilla) | 5 - 20 g |
| Live probiotic: *L. acidophilus* (Danisco HowAreU); *Bifidus* (Danisco) | 10 BCFU, total (1-100 BCFU, optionally) |
| Heat treated probiotic: *Lactobacillus plantarum* (House Wellness Immuno20 HL L137) | 10 mg |

**Table 11b**

| Membrane | |
|---|---|
| Ingredient | Amount per individual serving |
| Sodium Alginate | 0.01 - 0.4 g |
| β-glucan (Wellmune Biothera) | 250 mg |
| Water | 1.0 - 4.0 |
| Calcium lactate | < 0.5 g |
| Fruit/flavoring | 1.0-15 g |
| Sweetener | 0.25 - 1.0 g |
| Sodium citrate (optional) | 0.025 - 0.1 g |
| Stabilizer | 0.025 - 0.1 g |

In general, an edible transport vessel can be made as follows. In a pot, combine 15 g sodium alginate and into 985 g of mineral water, then heat over a low heat until it simmers. Mix until alginate is completely dissolved and solution has a uniform consistency. Let set at 4° C for 2 - 3 hours. Add sugar, stabilizer, flavorings, and other membrane components to a final concentration desired for the individual transport vessels, and mix to a uniform consistency. Prepare a 2% calcium bath by mixing 20 g of calcium lactate with 1 liter water. Dissolve completely. Blend yogurt and sugar to consistent texture, and add to a pastry bag, piping bag or similar device. Dip end of pastry bag into inner membrane alginate solution, and form small spheres of 1-2 inch diameter. Alternatively, frozen, preformed spheres of an encapsulated food composition (e.g., frozen yogurt or ice cream with probiotic) can be placed into the alginate solution, with or without a first calcium bath dip. Remove spheres from membrane alginate solution and place into calcium bath for 10-15 minutes. Remove spheres and dry the surface with absorbing paper. Store at 4° C or -20° C.

While certain combinations of membranes and inner compositions have been provided and described as being used together, other combinations are possible.

## Claims

1. A functional food composition, comprising:
an edible or potable substance;
a cross-linked edible membrane matrix fully encapsulating the edible or potable substance;
a probiotic;
and a prebiotic, with the proviso that the edible or potable substance and the matrix do not both contain the probiotic or both contain the prebiotic, wherein the matrix comprises the prebiotic and the edible or potable substance component comprises the probiotic, or
wherein the matrix comprises the probiotic and the edible or potable substance comprises the prebiotic.

2. The functional food composition of any one of claims 1, wherein the composition comprises a probiotic concentration from 1.0 to 100 billion colony forming units in an individual edible transport vessel.

3. The functional food composition of any one of claims 1-2, wherein the probiotic is an organism selected from the genera group consisting of *Aspergillus, Bacillus, Bacteroides, Bifidobacterium, Brettanomyces, Enterococcum, Kluyveromyces, Lactobacillus, Lactococcus, Leuconostoc, Pediococcus, Propionibacterium, Saccharomyces, Shewanella, Streptococcus, Torulaspora, Vagococcus,* and combinations thereof.

4. The functional food composition of any one of claims 1-3, wherein the probiotic is an organism comprising *Bacillus coagulans.*

5. The functional food composition of any one of claims 1-4, wherein the prebiotic is one of the group consisting of galacto-oligosaccharides, inulin, oligofructose, isomalto-oligosaccharides, lactulose, lactosucrose, transgalacto-oligosaccharides, soybean oligosaccharides, tagatose, xylo-oligosaccharides, and combinations thereof.

6. The functional food composition of any one of claims 1-5, wherein the composition comprises a prebiotic concentration from 1.0 to 30.0 grams in an individual edible transport vessel.

7. The functional food composition of any one of claims 1-6, wherein the matrix is comprised of an edible polymer charge cross-linked by multivalent ions, including cross-linking interactions between the edible particles and edible polymer or plurality of edible polymers via bridges formed by the multivalent ions.

8. The functional food composition of claim 1-7, wherein the matrix comprises a polysaccharide selected from the group consisting of a hydrocolloid, shellac, and fibers.

9. The functional food composition of any one of claims 1-8, further comprising additional edible particles in the cross-linked matrix.

10. A method of preparing a functional food composition, comprising the steps of:
a) providing an edible or potable substance, an edible polymer, a probiotic, and a prebiotic;
b) combining the edible or potable substance with the probiotic or the prebiotic;
c) encapsulating the edible substance with the probiotic or the prebiotic in an edible matrix that comprises the edible polymer and the probiotic or the prebiotic; with the proviso that the edible or potable substance and the matrix do not both contain the probiotic or both contain the prebiotic.

11. The method of claim 10, wherein the composition comprises a probiotic concentration from 1.0 to 100 billion colony forming units in an individual edible transport vessel.

12. The method of claim 10 or 11, wherein the probiotic is an organism comprising *Bacillus coagulans.*

## Patentansprüche

1. Funktionelle Nahrungsmittelzusammensetzung, umfassend:
eine essbare oder trinkbare Substanz;
eine vernetzte essbare Membranmatrix, die die essbare oder trinkbare Substanz vollständig einkapselt;
ein Probiotikum;
und ein Präbiotikum mit der Maßgabe, dass die essbare oder trinkbare Substanz und die Matrix nicht beide das Probiotikum enthalten oder beide das Präbiotikum enthalten, wobei (die Matrix das Präbiotikum und die essbare oder trinkbare Substanzkomponente das Probiotikum umfasst, oder
wobei die Matrix das Probiotikum umfasst und die essbare oder trinkbare Substanz das Präbiotikum umfasst.

2. Funktionelle Nahrungsmittelzusammensetzung nach einem der Ansprüche 1, wobei die Zusammensetzung eine Probiotikumkonzentration von 1,0 bis 100 Milliarden koloniebildenden Einheiten in einem einzelnen essbaren Transportgefäß umfasst.

3. Funktionelle Nahrungsmittelzusammensetzung nach einem der Ansprüche 1 bis 2, wobei das Probiotikum ein Organismus ist, ausgewählt aus der Gattungsgruppe bestehend aus *Aspergillus, Bacillus, Bacteroides, Bifidobacterium, Brettanomyces, Enterococcum, Kluyveromyces, Lactobacillus, Lactococcus, Leuconostoc, Pediococcus, Propionibacterium, Saccharomyces, Shewanella, Streptococcus, Torulaspora, Vagococcus* und Kombinationen davon.

4. Funktionelle Nahrungsmittelzusammensetzung nach einem der Ansprüche 1 bis 3, wobei das Probiotikum ein Organismus ist, der Bacillus coagulans umfasst.

5. Funktionelle Nahrungsmittelzusammensetzung nach einem der Ansprüche 1 bis 4, wobei das Präbiotikum zu der Gruppe gehört, die aus Galacto-Oligosacchariden, Inulin, Oligofructose, Isomalto-Oligosacchariden, Lactulose, Lactosucrose, Transgalacto-Oligosacchariden, Sojabohnen-Oligosacchariden, Tagatose, Xylo-Oligosaccharide und Kombinationen davon besteht.

6. Funktionelle Nahrungsmittelzusammensetzung nach einem der Ansprüche 1 bis 5, wobei die Zusammensetzung eine Präbiotikumkonzentration von 1,0 bis 30,0 g in einem einzelnen essbaren Transportgefäß umfasst.

7. Funktionelle Nahrungsmittelzusammensetzung nach einem der Ansprüche 1 bis 6, wobei die Matrix aus einer essbaren Polymerladung besteht, die durch mehrwertige Ionen vernetzt ist, die Vernetzungswechselwirkungen zwischen den essbaren Partikeln und essbarem Polymer oder mehreren essbaren Polymeren über Brücken, die durch die mehrwertigen Ionen gebildet werden, einschließt.

8. Funktionelle Nahrungsmittelzusammensetzung nach Anspruch 1 bis 7, wobei die Matrix ein Polysaccharid umfasst, das aus der Gruppe ausgewählt ist, bestehend aus einem Hydrokolloid, Schellack und Fasern.

9. Funktionelle Nahrungsmittelzusammensetzung nach einem der Ansprüche 1 bis 8, ferner umfassend zusätzliche essbare Partikel in der vernetzten Matrix.

10. Verfahren zur Herstellung einer funktionellen Nahrungsmittelzusammensetzung, umfassend die Schritte:
a) Bereitstellen einer essbaren oder trinkbaren Substanz, eines essbaren Polymers, eines Probiotikums und eines Präbiotikums;
b) Kombinieren der essbaren oder trinkbaren Substanz mit dem Probiotikum oder dem Präbiotikum;
c) Einkapseln der essbaren Substanz mit dem Probiotikum oder dem Präbiotikum in eine essbare Matrix, die das essbare Polymer und das Probiotikum oder das Präbiotikum umfasst; mit der Maßgabe, dass die essbare oder trinkbare Substanz und die Matrix nicht beide das Probiotikum oder beide das Präbiotikum enthalten.

11. Verfahren nach Anspruch 10, wobei die Zusammensetzung eine Probiotikumkonzentration von 1,0 bis 100 Milliarden koloniebildenden Einheiten in einem einzelnen essbaren Transportgefäß umfasst.

12. Verfahren nach Anspruch 10 oder 11, wobei das Probiotikum ein Organismus ist, das *Bacillus coagulaiis* umfasst.

## Revendications

1. Composition d'aliment fonctionnel comprenant
une substance comestible ou buvable ;
une matrice de membrane comestible réticulée encapsulant complètement la substance comestible ou buvable ;
un probiotique ;
et un prébiotique, sous réserve que la substance comestible ou buvable et la matrice ne contiennent pas toutes deux le probiotique ou ne contiennent pas toutes deux le prébiotique,
dans laquelle la matrice comprend le prébiotique et le composant de substance comestible ou buvable comprend le probiotique, ou
dans laquelle la matrice comprend le probiotique et la substance comestible ou buvable comprend le prébiotique.

2. Composition d'aliment fonctionnel selon la revendication 1, laquelle composition comprend une concentration de probiotique de 1,0 à 100 milliards d'unités formant des colonies dans un récipient de transport comestible individuel.

3. Composition d'aliment fonctionnel selon l'une quelconque des revendications 1 et 2, dans laquelle le probiotique est un organisme choisi parmi les groupes de genres constitués par *Aspergillus, Bacillus, Bacteroides, Bifidobacterium, Brettanomyces, Enterococcum, Kluyveromyces, Lactobacillus, Lactococcus, Leuconostoc, Pediococcus, Propionibacterium, Saccharomyces, Shewanella, Streptococcus, Torulaspora, Vagococcus,* et leurs combinaisons.

4. Composition d'aliment fonctionnel selon l'une quelconque des revendications 1 à 3, dans laquelle le probiotique est un organisme comprenant *Bacillus coagulans.*

5. Composition d'aliment fonctionnel selon l'une quelconque des revendications 1 à 4, dans laquelle le prébiotique est l'un du groupe constitué par les galacto-oligosaccharides, l'inuline, l'oligofructose, les isomalto-oligosaccharides, le lactulose, le lactosaccharose, les transgalacto-oligosaccharides, les oligosaccharides de soja, le tagatose, les xylo-oligosaccharides, et leurs combinaisons.

6. Composition d'aliment fonctionnel selon l'une quelconque des revendications 1 à 5, laquelle composition comprend une concentration de prébiotique de 1,0 à 30,0 grammes dans un récipient de transport comestible individuel.

7. Composition d'aliment fonctionnel selon l'une quelconque des revendications 1 à 6, dans laquelle la matrice est constituée d'une charge polymère comestible réticulée par des ions multivalents, comprenant des interactions de réticulation entre les particules comestibles et un polymère comestible ou une pluralité de polymères comestibles via des ponts formés par les ions multivalents.

8. Composition d'aliment fonctionnel selon l'une quelconque des revendications 1 à 7, dans laquelle la matrice comprend un polysaccharide choisi dans le groupe constitué par un hydrocolloïde, la gomme laque, et les fibres.

9. Composition d'aliment fonctionnel selon l'une quelconque des revendications 1 à 8, comprenant en outre des particules comestibles additionnelles dans la matrice réticulée.

10. Procédé de préparation d'une composition d'aliment fonctionnel, comprenant les étapes suivantes :
a) fourniture d'une substance comestible ou buvable, d'un polymère comestible, d'un probiotique, et d'un prébiotique ;
b) combinaison de la substance comestible ou buvable avec le probiotique ou le prébiotique ;
c) encapsulation de la substance comestible avec le probiotique ou le prébiotique dans une matrice comestible qui comprend le polymère comestible et le probiotique ou le prébiotique ;
sous réserve que la substance comestible ou buvable et la matrice ne contiennent pas toutes deux le probiotique ou ne contiennent pas toutes deux le prébiotique.

11. Procédé selon la revendication 10, dans lequel la composition comprend une concentration de probiotique de 1,0 à 100 milliards d'unités formant des colonies dans un récipient de transport comestible individuel.

12. Procédé selon la revendication 10 ou 11, dans lequel le probiotique est un organisme comprenant *Bacillus coagulans.*
